(19)

**Europäisches Patentamt**
**European Patent Office**
**Office européen des brevets**

(11) **EP 3 892 246 B1**

(12) **EUROPEAN PATENT SPECIFICATION**

(45) Date of publication and mention
of the grant of the patent:
**19.06.2024 Bulletin 2024/25**

(51) International Patent Classification (IPC):
***A61F 13/15*** *(2006.01)* ***B05C 5/02*** *(2006.01)*

(21) Application number: **21167447.8**

(52) Cooperative Patent Classification (CPC):
**A61F 13/15699;** B05C 5/0254

(22) Date of filing: **08.04.2021**

(54) **METHOD FOR APPLYING A POLYMERIC COMPOSITION AND ABSORBENT ARTICLES COMPRISING SUCH COMPOSITION**

VERFAHREN ZUM AUFBRINGEN EINER POLYMERZUSAMMENSETZUNG UND ABSORBIERENDE ARTIKEL MIT EINER SOLCHEN ZUSAMMENSETZUNG

PROCÉDÉ D'APPLICATION D'UNE COMPOSITION POLYMÈRE ET ARTICLES ABSORBANTS COMPRENANT UNE TELLE COMPOSITION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.04.2020 US 202063006934 P**

(43) Date of publication of application:
**13.10.2021 Bulletin 2021/41**

(73) Proprietor: **The Procter & Gamble Company Cincinnati, OH 45202 (US)**

(72) Inventors:
• **STRASEMEIER, John Andrew**
**Cincinnati, 45202 (US)**
• **TURNER, Robert Haines**
**Cincinnati, 45202 (US)**
• **LINDNER, Torsten**
**65824 Schwalbach am Taunus (DE)**
• **GIBSON, Fredrick William**
**Cincinnati, 45202 (US)**

(74) Representative: **P&G Patent Germany Procter & Gamble Service GmbH Sulzbacher Straße 40 65824 Schwalbach am Taunus (DE)**

(56) References cited:
**WO-A1-2014/085063     WO-A1-2020/076906
US-A1- 2014 296 811**

EP 3 892 246 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD

The present disclosure relates in a first aspect to a method for applying a polymeric composition having a Tensile Strength at Yield of from 0.5 MPa to 10 MPa in a fluid form to a substrate by a contact method which is different from conventional slot coating. The polymeric composition may be used to providing a texture to a substrate or to bond two substrates together. The polymeric composition may be applied in a pre-defined pattern. In a second aspect, the present disclosure is for an absorbent article comprising a substrate having the polymeric composition deposited thereon on a pattern, which is not a slot pattern. The substrate on which the polymeric composition is applied may in particular be a nonwoven.

BACKGROUND

Users, for example caregivers, rely on disposable absorbent articles to make their lives easier. Disposable absorbent articles, such as adult incontinence articles, diapers, and training pants are generally manufactured by combining several components. These components typically include a chassis comprising a liquid-permeable topsheet, a liquid-impermeable backsheet attached to the topsheet, an absorbent core located between the topsheet and the backsheet, and a plurality of bond areas holding the chassis together. When the disposable article is worn, the liquid-permeable topsheet is positioned next to the body of the wearer. The topsheet allows passage of bodily fluids into the absorbent core. The liquid-impermeable backsheet helps prevent leakage of fluids held in the absorbent core. The absorbent core generally is designed to have desirable physical properties, e.g. a high absorbent capacity and high absorption rate, so that bodily fluids can be transported from the skin of the wearer into the disposable absorbent article.

Frequently one or more components of a disposable absorbent article are bonded together. For example, hot melt adhesives have been used to bond individual layers of the chassis of the absorbent article, such as the topsheet and backsheet together. Hot melt adhesives have also been used to bond discrete components, such as fasteners and leg elastics or cuffs, to the article. The hot melt adhesive is often called a construction adhesive because it is used to help construct the absorbent article from individual components.

Common hot melt adhesives are made by combining polymer and additive components in a substantially uniform thermoplastic blend. Typical additives include tackifiers, plasticizers, and/or waxes, for example. While such formulations generally work, they can be costly and their performance properties can be improved. For example, tackifiers, which can comprise up to 65% of an adhesive formula, can be expensive and difficult to source. Therefore, there is a need for improved hot melt adhesives, such as substantially tackifier-free polymeric compositions, that offer (1) superior performance compared to tackifier-based hot melt adhesives (2) at a lower cost.

Several formulations have been accordingly proposed. For example, US214/0296811A1 (Bunnelle) discloses using a hotmelt comprising (i) an amorphous polyolefin composition and (i) a heterophase polyolefin comprising amorphous character and crystalline block for joining two absorbent article components. US2016/0053,149A1 (Herrlich et al., Clariant) discloses a ready-to-use hotmelt adhesive comprising at least 95 wt % of one or more polyolefin copolymer waxes produced using metallocene catalysts, where the polyolefin copolymer wax consists of propylene and one or more further monomers selected from ethylene and branched or unbranched 1-alkenes having 4 to 20 C atoms. More recently, polymer compositions having a Tensile Strength at Yield of from 0.5 MPa to 10 MPa have been proposed in US2020/0108167A1 ,filed on October 9, 2019, for making nonwoven-nonwoven bond. These polymeric compositions have the advantages of requiring no or little blending with other ingredients, which saves costs and increase purity.

However, one issue with nearly all these polymeric compositions is severe processability limitation with application at high line speeds via slot die nozzles. In the slot process, the nozzle is in direct contact with and drags over the substrate while the bonding agent (conventional adhesive or polymeric composition) is deposited onto it. The bonding agent leaves the application head via a slit with a typical width of 150 to 250 $\mu$m in machine direction.

Using such slot die nozzles, the inventors observed rapid build-up of the polymeric composition downstream of the slot die nozzle (phenomenon sometimes called "blobbing", "drooling" or "accretion"), when run at high line speed, most times nearly instantaneously, and the latest after about 2 minutes. This contamination causes line stops after short periods of running time. It may, in the worst case, lead to consumer-noticeable defects in the product if a larger accumulation ("blob") of the solidified bonding agent falls off the nozzle onto the substrate and is included in the product.

Contact applications are generally preferred over spray applications for many usages because the latter always require the supply with heated air on the production lines (higher production cost). They also necessitate more frequent cleaning (more downtime) of the line as not all of the sprayed adhesive ends up precisely in the product but a small fraction always "lands" on equipment parts, carried by the air, where it accumulates over time. Also, spray applications which can deliver a spiral adhesive pattern or random fibrous pattern (e.g. known as commercial solutions sold under name Omega®, Summit®, Control Coat®, Curtain Coat® or Signature® applications) only allow for comparably "open" adhesive application patterns and do not allow for completely covering or sealing the complete surface or a defined portion of the surface of the primary substrate with a coherent "film" of adhesive. The patterns generated by spray applications are defined by thin adhesive strands, which are generated during the spraying process and whose diameters typically range between 5 and 20 μm. On a microscopic level, these thin adhesive strands do by purpose not completely cover the surface of the substrate but leave adhesive free zones in between. A typical random fibrous pattern (as e.g. accessible via the Control Coat® application) is characterized by adhesive free zones on the surface on a sub-millimeter scale. The fiber diameter of a typical spun nonwoven, for comparison, ranges between 15 and 20 μm, so that a zone of a spun nonwoven covered with a spray pattern can stay permeable to air or liquid. A complete coverage may, however, be desirable for some applications, like e.g. sealing bonds (like the C-wrap bond in the core bag). Spray applications do not allow, either, - unlike contact applications - for patterns of more complex geometry not oriented in the machine direction, such as discrete pattern elements like squares, dots, rectangles.

Bead application is an example of a non-contact adhesive application method which does not operate with process air (unlike a spray application). In bead, the adhesive is extruded through a millimeter large orifice and falls by gravity onto the substrate as a continuous bead of adhesive. The adhesive pattern thus laid down can have a width of around a millimeter (as it has not been stretched via air) and can allow for effective sealing, but bead applications cannot be applied intermittently and there are limitations to the line speed with which they can be used. In any case, these adhesive beads can only be laid down in machine direction.

There is therefore a need for an economic method that enables the contact application of polymeric compositions as described above.

SUMMARY

In a first aspect, the invention is for a method for applying a polymeric composition from a slot die applicator to a substrate as indicated in the claims. The polymeric composition has a Tensile Strength at Yield of from 0.5 MPa to 10 MPa according to the Tensile Strength Test Method described herein. The slot die applicator includes a slot opening, a first lip, and a second lip, the slot opening located between the first lip and the second lip.

The method of the invention comprises the steps of engaging the substrate with a substrate carrier such that the first surface is in face-to-face contact with the substrate carrier and the second surface faces outward from the substrate carrier, and continuously advancing the substrate past the first lip, the slot opening, and the second lip of the slot die applicator while the first surface of the substrate is disposed on a substrate carrier, and discharging the polymeric composition from the slot opening of the slot die applicator onto a second surface of the substrate.

While not wishing to be bound by theory, the inventors believe that the present method allows for a very effective direct control of the coating gap (i.e. the gap between the slot opening and the substrate) via the substrate carrier that maintains the substrate at an optimal position distance to the slot opening. This was found to enable the efficient application of the polymeric composition claimed without significant blobbing even at high speed. The method may be typically carried out at a substrate speed of more than 2 m/s, in particular of more than 3 m/s, or even of more than 4 m/s.

In the present invention, the coating gap is directly controlled by fixed equipment parts, i.e. the slot die applicator and the substrate carrier, as opposed to indirect control via the web tension only as is used in conventional slot coating. While the claimed method involves some tension of the web during the application of the polymeric composition, a lower web tension can be used relative to slot die conventional processes. Without this direct control, the inventor believes that the coating gap is subjected to larger variations that can create the anomaly described above as blobbing. In the present invention, the substrate may be subject to a tension at the point of application of less than 10 N/m (Force per unit width), in particular from about 1 N/m to about 6 N/m. A lower tension value can provide for a more reliable application process, especially a more accurate deposition pattern.

The substrate carrier used may take different forms. In simple executions, the substrate carrier may be for example a flat or curved plate, or a smooth roll. This allows for low complexity and low capital cost, as will described further below.

In a more sophisticated approach, the substrate carrier is a patterned roll, the roll being advantageously driven by a motor. This method allows for applying the polymeric composition from a slot die applicator to the substrate in a pattern, the substrate having a first surface disposed opposite of a second surface and an unconstrained caliper, Hs. This method may in particular comprise the steps of:

- continuously advancing the substrate in a machine direction;
- engaging the substrate with a substrate carrier such that the first surface is in face-to-face contact with the substrate carrier and the second surface faces outward from the substrate carrier, wherein the substrate carrier comprises a non-compliant support surface and a pattern element, the pattern element including a pattern surface, wherein the pattern element extends away from the non-compliant support surface to define a first minimum distance, R1, between the pattern surface and the non-compliant support surface;

- positioning the substrate carrier adjacent the slot die applicator to define a minimum distance, Hg, between the pattern surface of the pattern element and the first lip and the second lip that is less than the unconstrained caliper, Hs, of the substrate;
- advancing the second surface of the substrate past the slot die applicator while the first surface of the substrate is disposed on the substrate carrier;
- intermittently deflecting the pattern surface toward the non-compliant support surface such to define a second minimum distance, R2, between the pattern surface and the non-compliant surface, wherein R2 is less than R1, by advancing the substrate and the pattern element past the first lip, the slot opening, and the second lip of the slot die applicator while the first surface of the substrate is disposed on the substrate carrier; and
- discharging fluid from the slot opening of the slot die applicator onto the second surface of the substrate.

Using a patterned substrate carrier enables the creation of application patterns having any desired shapes, which may be a regular geometric pattern, or a non-regular pattern, or a combination thereof. In particular, the pattern is not limited to stripes oriented in machine direction as is the case for standard slot coating process. The pattern may in particular comprise discrete or continuous elements having a dimension in machine direction which is less than 10 mm, for example from 1 mm to 10 mm.

In a second aspect, the invention is for absorbent article comprising a substrate, in particular a nonwoven material, and a polymeric composition applied on the substrate according to a contact application, the polymeric composition having a Tensile Strength at Yield of from 0.5 MPa to 10 MPa according to the Tensile Strength Test Method described herein, wherein the application pattern does not consist solely of one or more stripes oriented in machine direction. The absorbent article may comprise any conventional components, in particular a topsheet, a backsheet, an absorbent core and an acquisition layer. The substrate may be any layers of the absorbent article, but in particular a nonwoven. The article may thus comprise two layers, one and advantageously both being a nonwoven, bonded by a pattern of the polymeric composition described herein. The application pattern may in particular comprise continuous and/or discrete pattern elements having a dimension in machine direction of less than 10 mm. The application pattern may also in particular comprise at least one cross-direction oriented stripe. Such stripes may be for example used to form core wrap end seals of the absorbent core.

In a further aspect, the polymeric composition used in the present invention may comprise less than 5 % of a tackifier, by weight of the polymeric composition. The polymeric composition may also comprise as main component a propylene butene copolymer, a polypropylene homopolymer, a propylene ethylene copolymer, or a mixture thereof.

BRIEF DESCRIPTION OF THE DRAWINGS

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:

Figure 1 is a perspective view of a fluid application apparatus positioned adjacent to an advancing substrate.

Figure 1A is a side view of a fluid application apparatus depositing fluid onto an advancing substrate in a first example pattern.

Figure 1B is a side view of a fluid application apparatus depositing fluid onto an advancing substrate in a second example pattern.

Figure 1C is a side view of a fluid application apparatus depositing fluid onto an advancing substrate in a third example pattern.

Figure 1D is a side view of a fluid application apparatus depositing fluid onto an advancing substrate in a fourth example pattern.

Figure 2A is a perspective view of an embodiment of a substrate carrier including a pattern roller having a continuous base surface and a plurality of pattern surfaces.

Figure 2B is a detailed cross-sectional view of the substrate carrier shown in Figure 2A taken along the line 2B-2B.

Figure 2C is a top side view of a substrate showing a first example adhesive pattern thereon.

Figure 3A is a perspective view of an embodiment of a substrate carrier including a pattern roller having a continuous pattern surface and plurality of base surfaces.

Figure 3B is a detailed cross-sectional view of the substrate carrier shown in Figure 3A taken along the line 3B-3B.

Figure 3C is a top side view of a substrate showing a second example adhesive pattern thereon.

Figure 4 is a schematic cross-sectional side view of an example substrate carrier.

Figure 4A1 is a detailed view of the substrate carrier of Figure 4 including a compliant pattern element and a compliant base layer connected with a base roll.

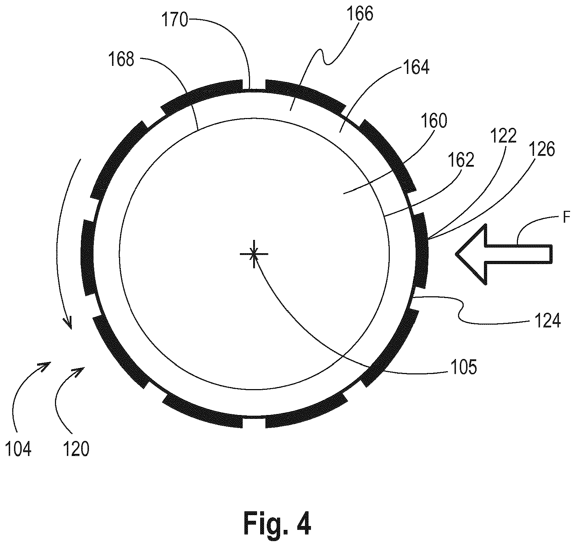

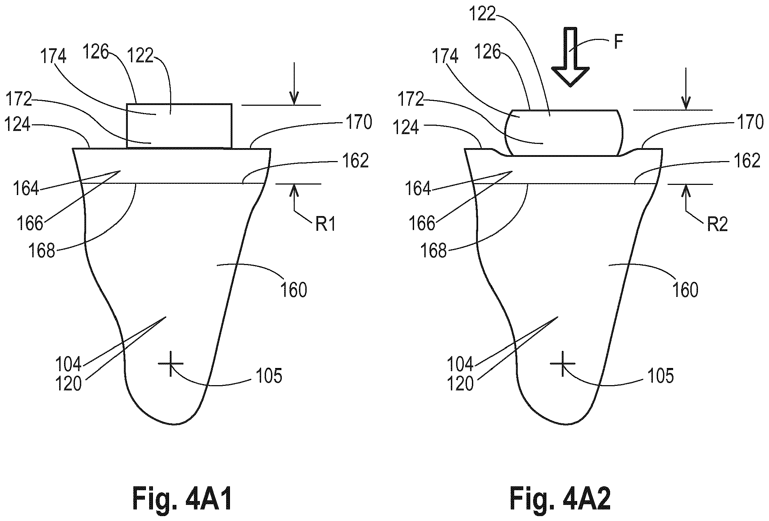

Figure 4A2 is a detailed view of the pattern surface of the pattern element from Figure 4A1 deflected by a force or forces applied to the pattern surface.

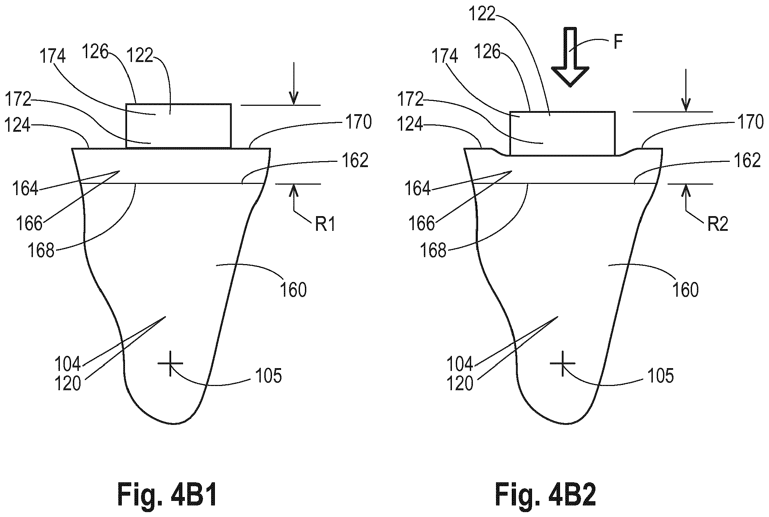

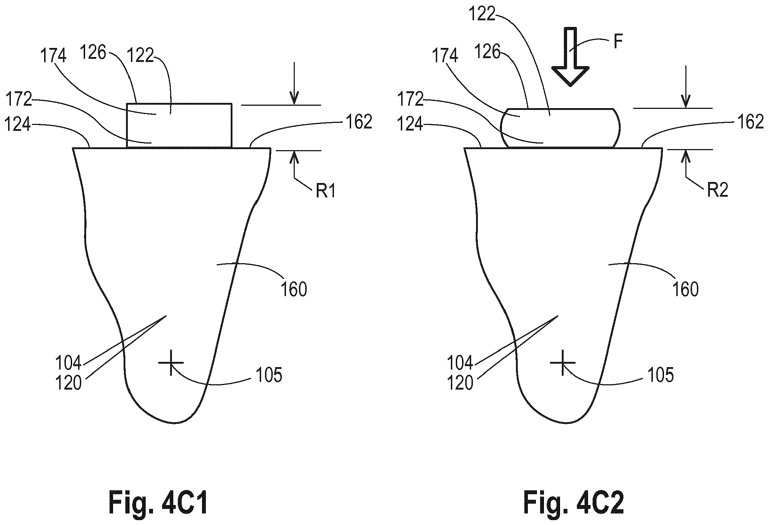

Figure 4B1 is a detailed view of the substrate carrier of Figure 4 including a non-compliant pattern element and a compliant base layer connected with a base roll.

Figure 4B2 is a detailed view of the pattern surface of the pattern element from Figure 4B1 deflected by a force or forces applied to the pattern surface.

Figure 4C1 is a detailed view of the substrate carrier of Figure 4 including a compliant pattern element connected with a base roll.

Figure 4C2 is a detailed view of the pattern surface of the pattern element from Figure 4C1 deflected by a force or forces applied to the pattern surface.

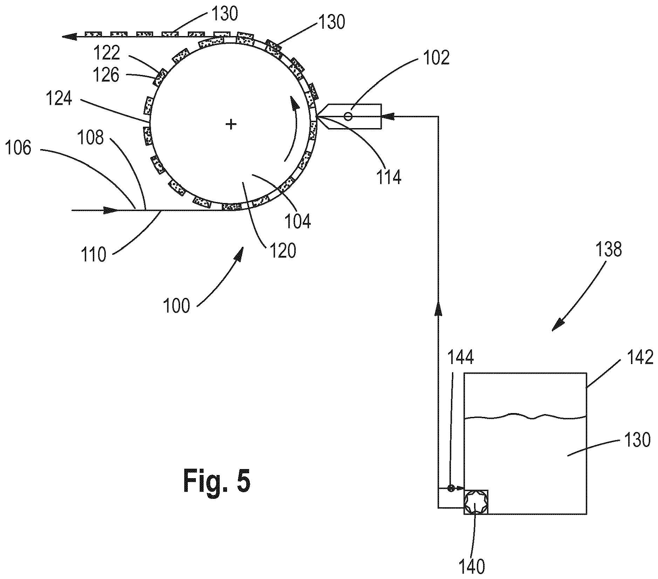

Figure 5 is a schematic cross-sectional side view of a fluid application apparatus.

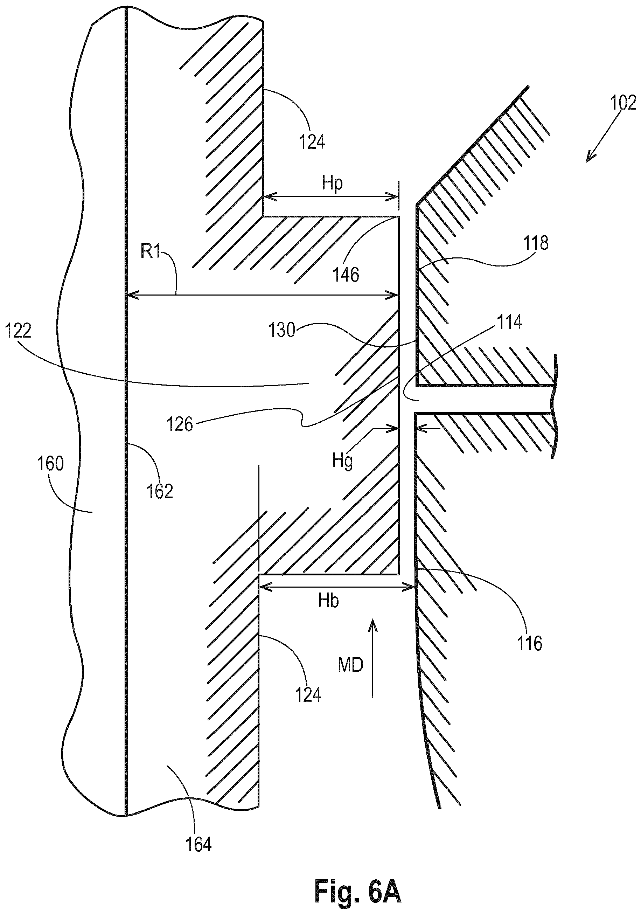

Figure 6A is a detailed cross-sectional view of the substrate carrier of Figure 5 without the substrate wherein the pattern surface of a pattern element is adjacent a first lip, a second lip, and slot opening of the slot die applicator.

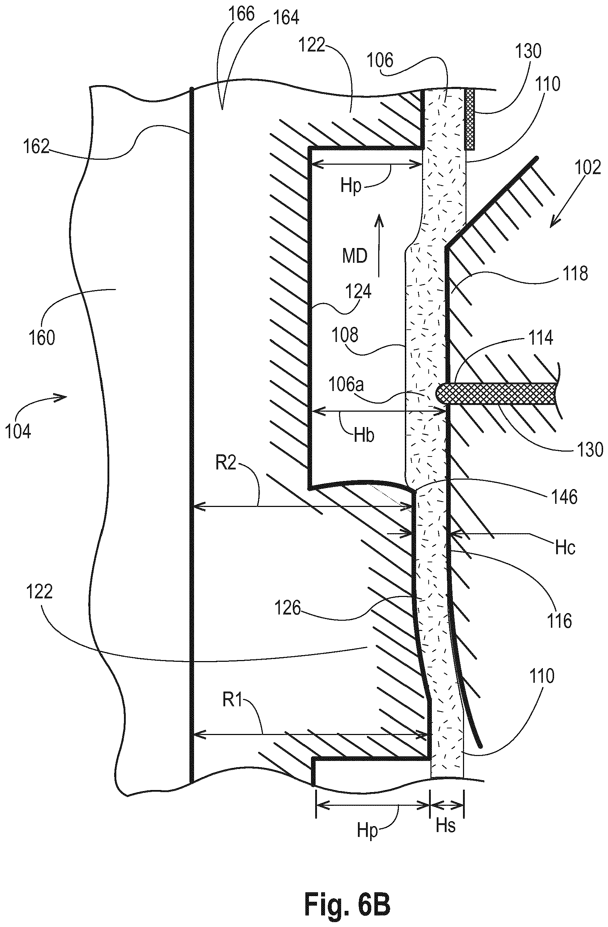

Figure 6B is a detailed cross-sectional view of a substrate carrier and a substrate advancing past a slot die applicator and showing the substrate between a slot opening of the slot die applicator and an advancing base surface.

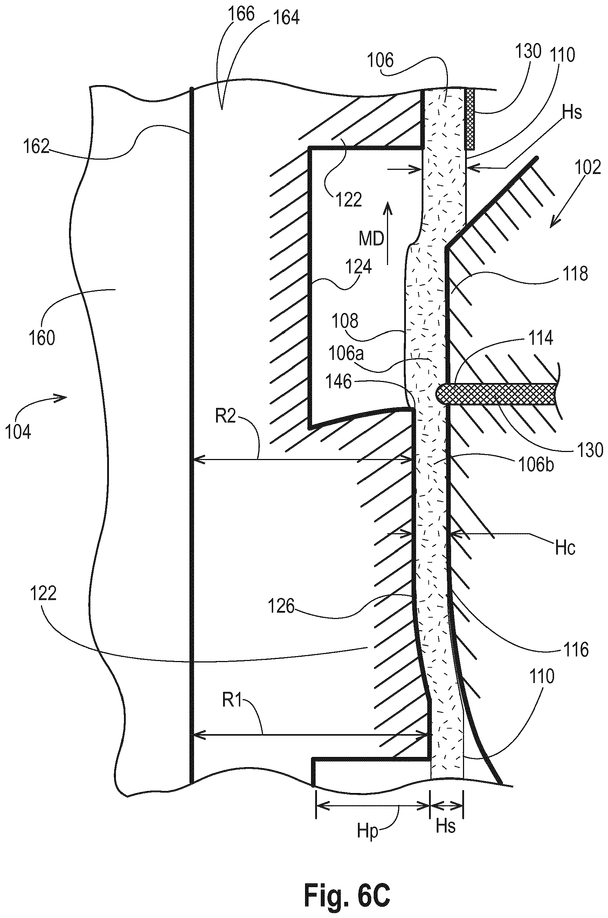

Figure 6C is a detailed cross-sectional view of the substrate carrier and substrate of Figure 6B wherein the base surface is advancing past the slot opening of the slot die applicator such that the substrate is between the slot opening of the slot die applicator and a leading edge of an advancing pattern surface.

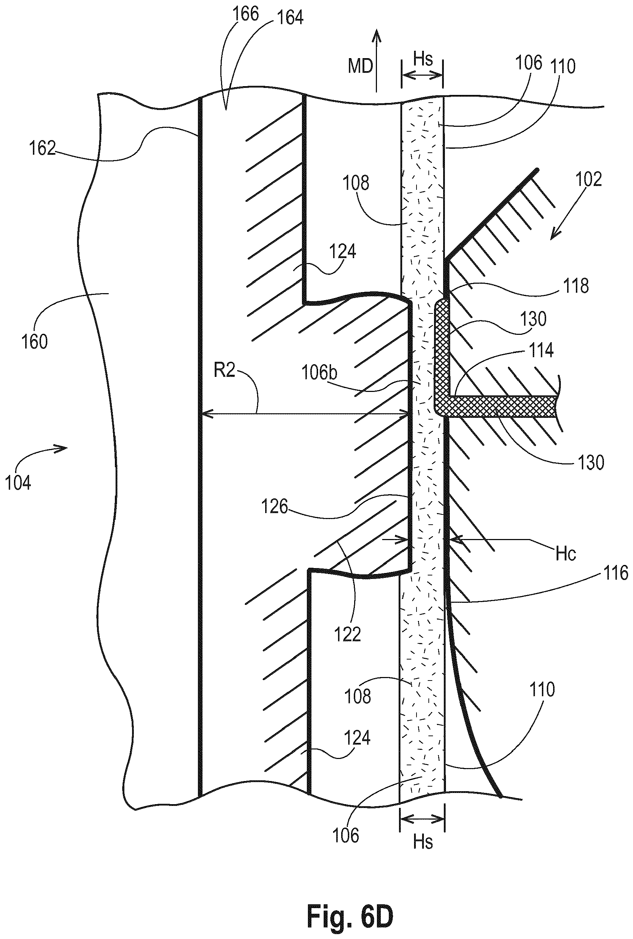

Figure 6D is a detailed cross-sectional view of the substrate carrier and substrate of Figure 6C wherein the base surface has advanced past the slot opening of the slot die applicator such that the substrate is between the slot opening of the slot die applicator and an advancing pattern surface.

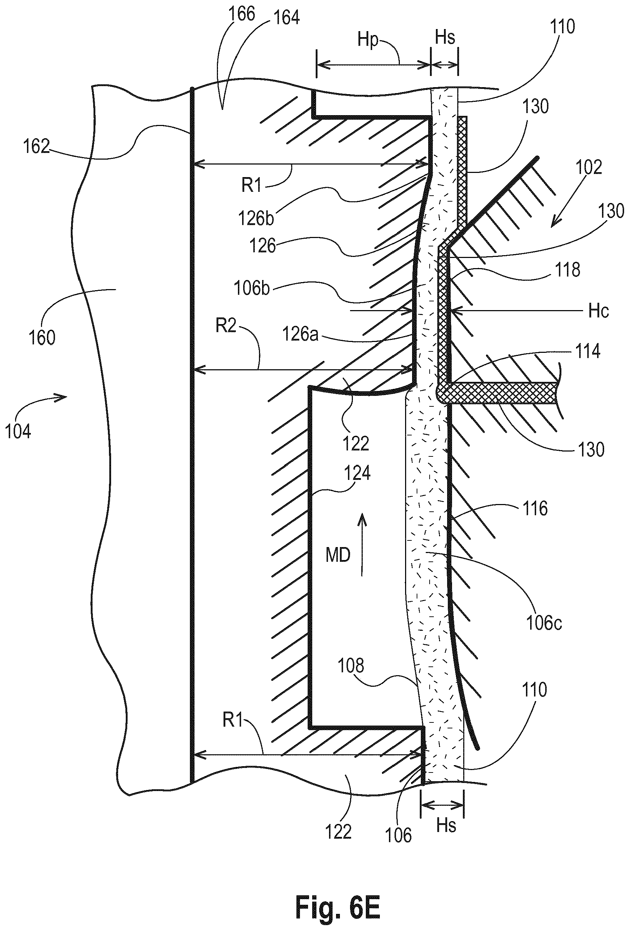

Figure 6E is a detailed cross-sectional view of the substrate carrier and substrate of Figure 6D wherein the pattern surface has advanced past the slot opening of the slot die applicator.

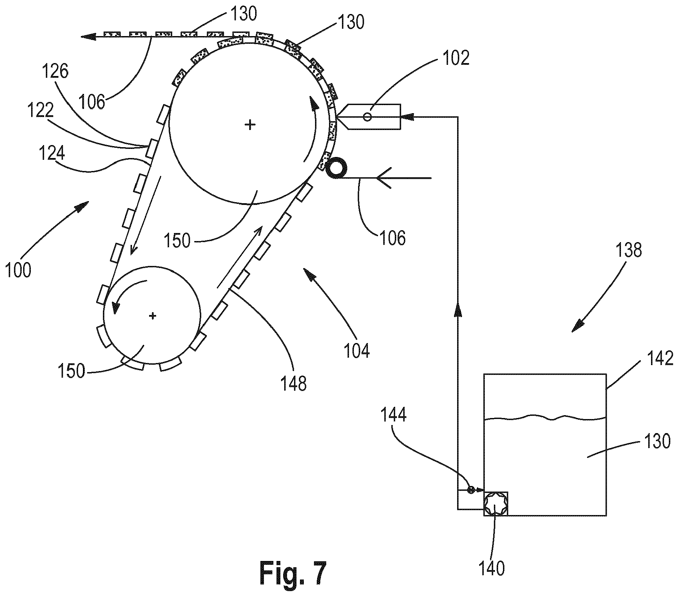

Figure 7 is a schematic cross-sectional side view of an embodiment of a fluid application apparatus with a substrate carrier including a pattern belt.

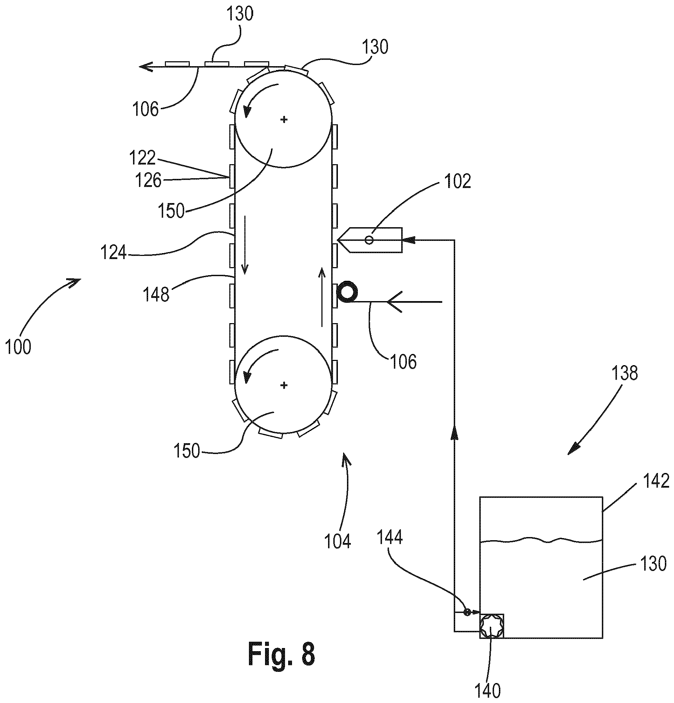

Figure 8 is a schematic cross-sectional side view of another embodiment of a fluid application apparatus with a substrate carrier including a pattern belt.

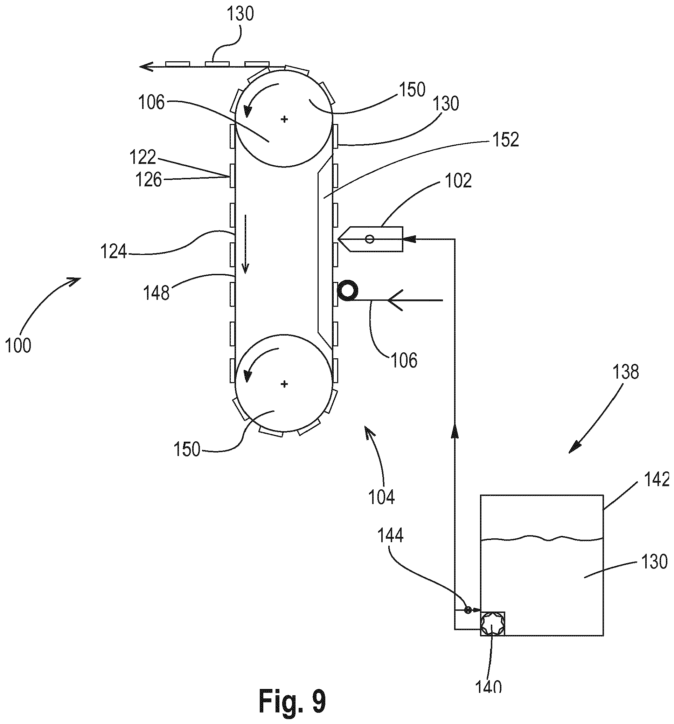

Figure 9 is a schematic cross-sectional side view of another embodiment of a fluid application apparatus with a substrate carrier including a pattern belt and a backup plate.

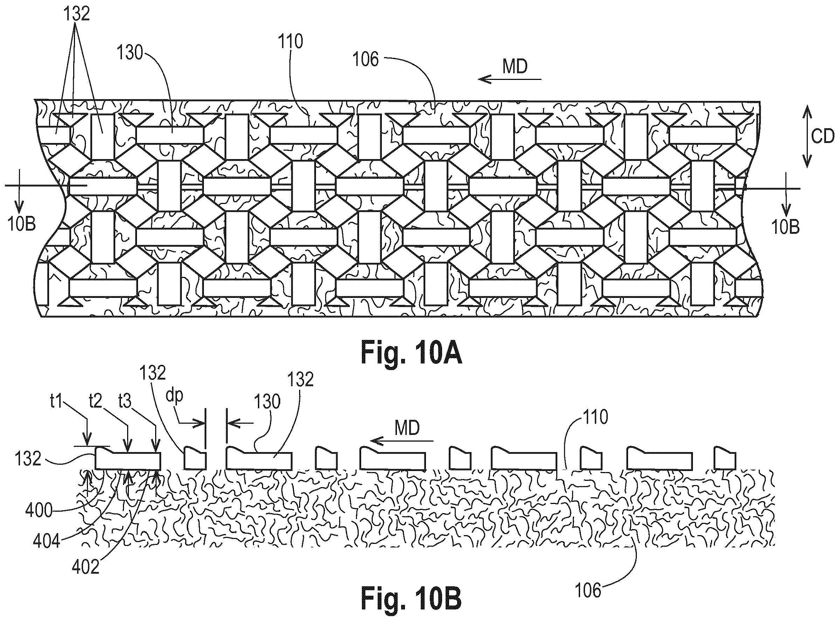

Figure 10A is a top plan view of a fluid applied in a pattern to a substrate.

Figure 10B is a cross sectional view of the substrate and fluid shown in Figure 10A taken along line 10B-10B.

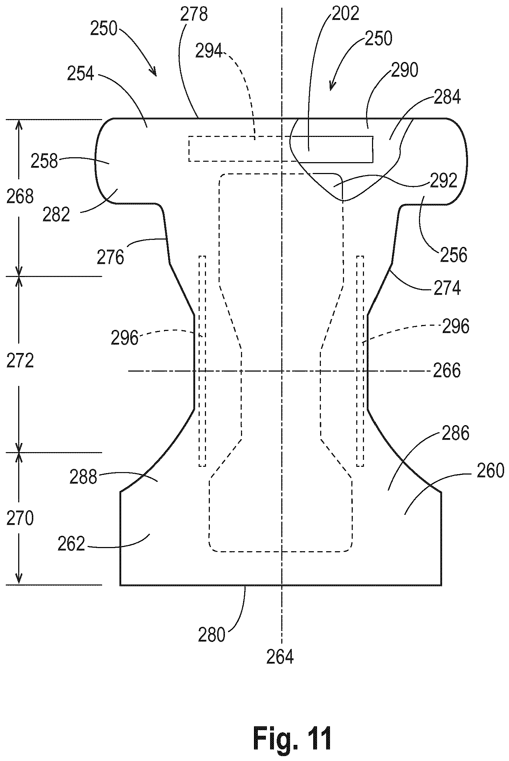

Figure 11 is a top plan view of a disposable absorbent article.

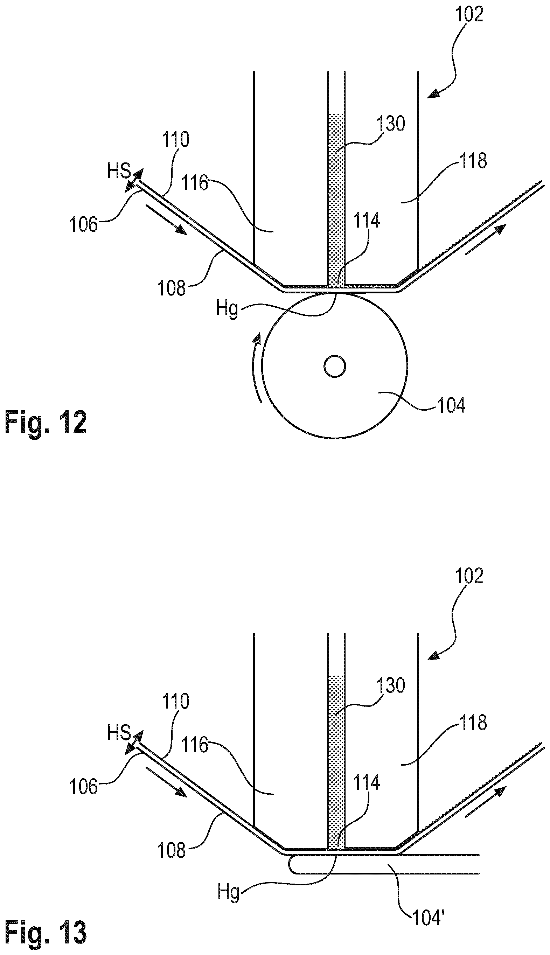

Fig. 12 and Fig. 13 schematically illustrate application devices and methods according to the invention.

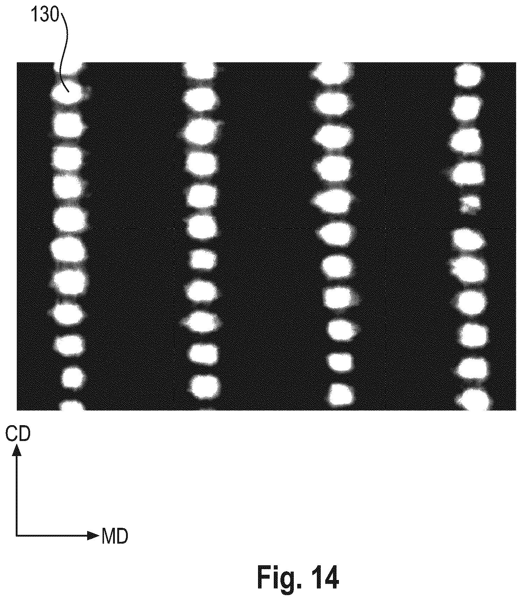

Fig. 14 shows a UV picture of an adhesive pattern obtained by the invention.

DETAILED DESCRIPTION

Term explanations

The following term explanations may be useful in understanding the present disclosure:
"Absorbent article" is used herein to refer to consumer products whose primary function is to absorb and retain soils and wastes. Non-limiting examples of absorbent articles include diapers, training pants, pull-on pant-type diapers, refastenable diapers or pant-type diapers, incontinence briefs and undergarments, diaper holders and liners, feminine hygiene garments such as panty liners, absorbent inserts, and the like.

"Diaper" is used herein to refer to an absorbent article generally worn by infants and incontinent persons about the lower torso.

The term "disposable" is used herein to describe absorbent articles which generally are not intended to be laundered or otherwise restored or reused as an absorbent article (e.g., they are intended to be discarded after a single use and may also be configured to be recycled, composted or otherwise disposed of in an environmentally compatible manner).

The term "disposed" is used herein to mean that an element(s) is formed (joined and positioned) in a particular place or position as a macro-unitary structure with other elements or as a separate element joined to another element.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

The term "substrate" is used herein to describe a material which is primarily two-dimensional (i.e. in an XY plane) and whose thickness (in a Z direction) is relatively small (i.e. 1/10 or less) in comparison to its length (in an X direction) and width (in a Y direction). Non-limiting examples of substrates include a layer or layers or fibrous materials, films and foils such as plastic films or metallic foils that may be used alone or laminated to one or more web, layer, film and/or foil. As such, a web is a substrate.

The term "nonwoven" refers herein to a material made from continuous (long) filaments (fibers) and/or discontinuous (short) filaments (fibers) by processes such as spunbonding, meltblowing, and the like. Nonwovens do not have a woven or knitted filament pattern.

The term "machine direction" (MD) is used herein to refer to the direction of material flow through a process. In addition, relative placement and movement of material can be described as flowing in the machine direction through a process from upstream in the process to downstream in the process.

The term "cross direction" (CD) is used herein to refer to a direction that is generally perpendicular to the machine direction.

The term "unconstrained caliper" refers to the caliper of the substrate measured according to Edana WSP 120.1 (05), with a circular presser foot having a diameter of $25.40 \pm 0.02$ mm and an applied force of 2.1 N (i.e. a pressure of $4.14 \pm 0.21$ kPa is applied).

The term "compliant" refers to any material with a durometer hardness of 90 or less as measured according to ASTM International Designation: D2240-05 (Reapproved 2010) for Type M durometers.

The term "non-compliant" refers to any material with a hardness value greater than 100 HRBW as defined on the Rockwell B Scale in the American National Standard Designation.

The term "polymeric composition" refers to a composition comprising at least 50 % by weight of one or more polymers, by weight of the polymeric composition.

The term "contact application" refers to a method where a composition is applied directly from a nozzle onto a moving substrate, whereby the nozzle is in direct contact with the substrate and typically drags over it during the application process. This excludes (i) spray applications in which process air is used to stretch the composition into thinner diameter fibers from the nozzle, and control their deposition pattern to form e.g. a series of overlapping spirals, or an elongated serpentine line without overlaps, or distribute these fibers more or less randomly, and (ii) bead applications (a filament or strand of the composition is extruded through an orifice and is deposited by gravity onto the substrate without involvement of air, i.e. without further stretching and diameter reduction or distribution into the cross direction).

Introduction

In its broadest aspect, the present disclosure involves methods and apparatuses utilizing continuous substrates for manufacturing articles, and more particularly, methods and apparatuses for applying compositions that have been heated to become fluid onto an advancing substrate. The fluid application apparatus includes a slot die applicator and a substrate carrier. The slot die applicator includes a slot opening, a first lip, and a second lip, the slot opening located between the first lip and the second lip. And the substrate carrier is adapted to advance the substrate past the slot die applicator as the slot die applicator discharges adhesive onto the substrate. In operation, when the first surface of the substrate is disposed on the substrate carrier, the substrate carrier advances the second surface of the substrate past the slot opening of the slot die applicator.

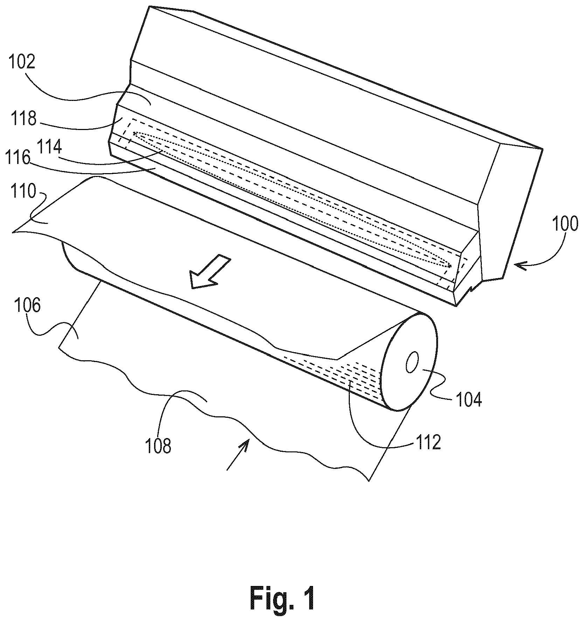

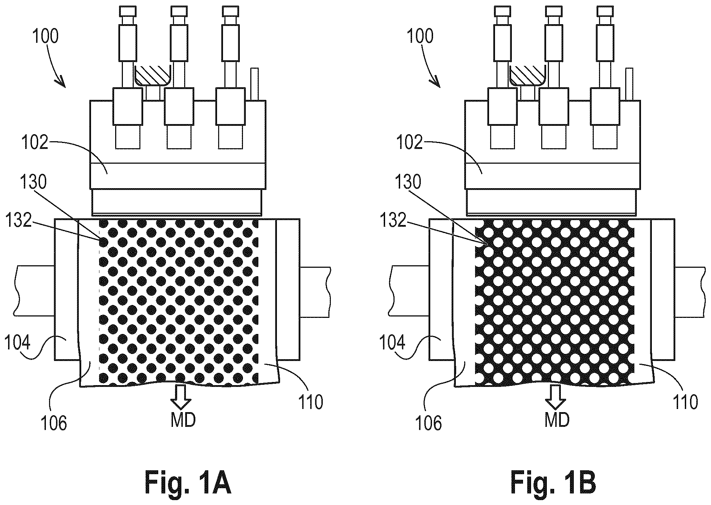

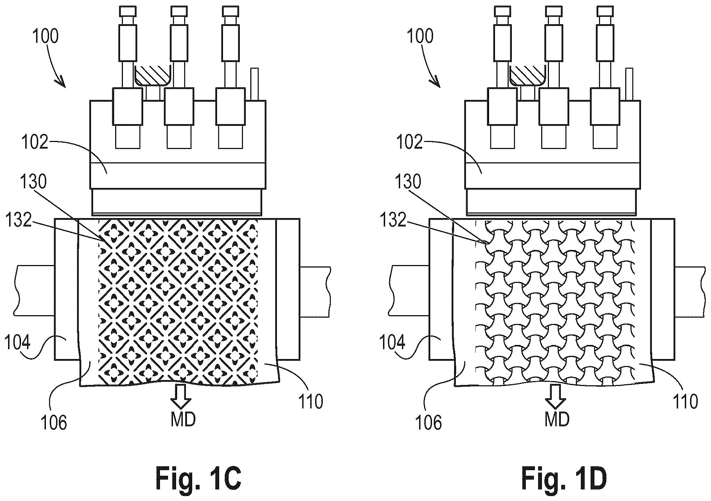

Particular embodiments of the apparatuses and methods disclosed herein provide for the application of viscous fluids, such as adhesives, in pre-determined patterns to an advancing substrate. The disclosure below relating to the apparatus as disclosed in Figure 1 to Figure 10 is generally identical to the disclosure in previous patent application WO2014/085063 (Brown et al., P&G). However, the present invention method is not limited to an application and may be used for simpler applications such as machine-direction oriented stripes, as will be briefly discussed in relation with Fig. 12-13 further below.

The polymeric compositions, including various examples, that are applied by the method of the present invention are described in a second part of the description further below.

General disclosure

Fig. 12 describes schematically a first execution of the method of the invention. As illustrated, the polymeric composition 130 is discharged in a fluid form from a slot die applicator 102 to a substrate 106. The slot die applicator 102 includes a slot opening 114, a first lip 116, and a second lip 118, the slot opening 114 is located between the first lip 116 and the second lip 118. The substrate 106 has a first surface 108 disposed opposite of a second surface 110 and an unconstrained caliper, Hs. The slot die applicator may also include a comb shim within the slot opening so that the polymeric composition is distributed according to a series of regions extending in cross-machine direction.

As illustrated, the method comprises the steps of:

- continuously advancing the substrate 106 in a machine direction;
- engaging the substrate 106 with a substrate carrier 104;
- positioning the substrate carrier 104 adjacent the slot die applicator 102 to define a minimum distance, Hg, between the surface of the substrate carrier and the first lip 116 and the second lip 118, that is substantially same or less than the unconstrained caliper, Hs, of the substrate;

- advancing the second surface 110 of the substrate 106 past the first lip 116, the slot opening 114, and the second lip 118 of the slot die applicator 102 while the first surface 108 of the substrate 106 is disposed on the substrate carrier 104; and
- discharging the polymeric composition 130 from the slot opening 114 of the slot die applicator 102 onto the second surface 110 of the substrate 106.

The substrate carrier illustrated in Fig. 12 has the form of a roll. Various type of rolls maybe used in the present invention. In a simple execution, the roll may be a smooth roll, for example of stainless steel or hard plastic such as polypropylene. In this example, the roll does not have a pattern at its surface, and the slot die applicator may advantageously include a comb shim within the slot opening so that the polymeric composition is distributed according to a series of regions extending in cross-machine direction. This results then in a series of longitudinal stripes being deposited on the substrate. The roll may be driven by an external motor (not shown) to help drive the substrate at the desired speed, but it may also be nondriven so that it is put in movement by friction with the substrate. In a preferred embodiment, the roll is however patterned, as will be discussed in relation with Figs. 1-10, so that a desired pattern may be applied on the substrate. The roll is therefore preferably driven to have a circumferential speed matching the substrate speed.

Fig. 13 illustrates an alternative example of substrate carrier embodied by a plate, which may be flat as represented, but may also be partially or completely curved. The surface of the plate guiding the substrate may be smooth, in which case the slot die applicator may be fitted with a distribution shim as discussed previously for the smooth roll, or the plate may also comprise a plurality of ridges oriented in machine direction along which the polymeric composition may be distributed onto the substrate.

As indicated previously, it is believed the superiority of the present method is due to the effective direct control of the coating gap (i.e. the gap between slot and substrate) via a substrate carrier (e.g. a roll) disposed at a controlled distance of the slot die applicator. Standard slot applications only allow for an indirect control of the coating gap via control of the web tension of the substrate with tension idlers. In conventional slot applications the web drags over the substrate. For better tension control, the substrate is often led over two idlers and dives to some extent into the substrate (with a defined depth of engagement). Thereby, the substrate is partly wrapped around the nozzle. A higher web tension is advantageous but there are limits to this approach, particularly at high line speeds. This indirect gap control has limitations as it is subjected to fluctuations due to (i) basis weight variations in the nonwoven and (ii) the irregular tearing action of the adhesive on a microscopic level when leaving the nozzle. The tearing action is due to a large speed mismatch between the web, which moves at high line speed (typically more than 2 m/s) beneath the nozzle, and the adhesive which leaves the slot nozzle with a typically 10x lower linear speed. So, the adhesive is accelerated by a factor of 10 when it gets into contact with the web.

Thereby, in conventional direct contact methods, the adhesive is normally torn into irregular splotches of a length in machine direction of 10 to 100 $\mu$m, rather than being applied as a uniform thin film. The stochastic variations in both the nonwoven basis weight and the adhesive application on a microscopic level, set limits to an effective indirect gap control. This is particularly true if the bonding agent has a too high viscosity at the shear rate with which the bonding agent leaves the nozzle (typically 1,000 to 10,000 s$^{-1}$ dependent on line speed and nozzle geometry). The polymeric compositions of the invention have typically higher viscosities at these shear rates as the shear thinning (decrease of shear viscosity with increasing shear rate) starts only at higher shear rates than observed with conventional adhesives. Therefore, the polymeric filler compositions are less able to penetrate the web when they leave the nozzle and stay on the surface of the web. So, the blobbing is caused by local gap opening and massively increased local coating weight - these larger "blobs" will then build up downstream of the nozzle. The local gap opening happens whenever the tension of the web is, due to the above described fluctuations, insufficient to compensate the force of the fluid which exits the coater.

In the present invention, a carrier substrate such as a driven roll is positioned in a fixed relationship with the nozzle which can be precisely adjusted. The substrate is engaged between the carrier substrate and the nozzle at the point of application of the polymeric composition. In conventional slot applications, the substrate is typically led over two idlers which are positioned on each side of the slot opening. Thereby, there is only a partial contact with the nozzle and relatively high web tension is required to compensate the force of the fluid exiting the nozzle. However there are limits to this conventional approach, particularly at high line speeds.

The present invention may use a flexographic sleeve provided with a pattern affixed on the surface of the roll. The roll takes up the fluid polymeric composition leaving the nozzle only at the protrusions on this sleeve, which enables the creation of intricate patterns, for example comprising discrete pattern elements of any shape, such as cross-direction or machine-direction oriented stripes with a short intermittent length, or artwork comprising curves lines or logos etc... A lower web tension is advantageous to obtain sharply resolved patterns. This advantageous embodiment will be now be discussed in relation to Figs. 1-10 in the next pages.

Patterned application (Figs. 1-10)

Figure 1 shows a perspective view an embodiment of an apparatus 100 for applying adhesives to a substrate (Figs. 1-11 are the same as previously disclosed in WO2014/085063). The apparatus 100 includes a slot die applicator 102 and a substrate carrier 104. As shown in Figure 1, a substrate 106 is advancing in a machine direction and is partially wrapped around the substrate carrier 104. More particularly, the substrate 106 includes a first surface 108 disposed opposite a second surface 110. And the first surface 108 of the substrate 106 is disposed on an outer surface 112 of the substrate carrier 104 while the second surface 110 of the substrate 106 advances past the slot die applicator 102. As discussed in more detail below, the second surface 110 of the substrate 106 advances past the slot die applicator 102 and the polymeric composition in fluid form is transferred from the slot die applicator 102 onto the second surface of the substrate. The application is preferably taking place in a pattern that is substantially the same as a pattern defined on the outer surface 112 of the substrate carrier 104. As discussed in more detail below, the substrate carrier 104 may be configured in various ways to deposit fluid 130 discharged from a slot die applicator 102 onto a substrate 106 in various different patterns, such as shown for example in Figures 1A through 1D.

It is to be appreciated that the slot die applicator 102 shown in Figure 1 is a generic representation of a device that is used to apply adhesive to the substrate 106. The slot die applicator includes a slot opening 114, a first lip 116, and a second lip 118. The first lip 116 may also be referred to herein as an upstream die lip, and the second lip 118 may also be referred to herein as a downstream die lip. The slot opening 114 is located between the first lip 116 and the second lip 118. Adhesive or other fluid may be discharged from the slot opening 114 onto the second surface 110 of the substrate 106 as the substrate carrier 104 advances the substrate past the first lip 116, slot opening 114, and second lip 118 of the slot die applicator 102. As discussed in more detail below, the substrate 106 is also intermittently compressed between the slot die applicator 102 and substrate carrier 104 as the substrate 106 advances past the slot die applicator 102. It is to be appreciated that various forms of slot die applicators may be used herein to apply adhesive or other fluids to an advancing substrate according to methods and apparatuses. For example, U.S. Patent No. 7,056,386 provides a description of slot die applicators that may be used. Other examples of commercially available slot die applicators include Nordson Corporation's EP11 Series of Slot Die Applicators and ITW Dynatec Gmbh's APEX Series of Slot Die Auto Adhesive Applicators.

As discussed in more detail below, the substrate carrier may include a base surface and a pattern element. The pattern element includes a pattern surface and protrudes outward from the base surface. As such, in substrate carriers configured with a base surface, the pattern surface and the base surface are separated by a distance, $H_p$. In addition, the substrate carrier is positioned adjacent the slot die applicator to define a minimum distance, $H_g$, between the pattern surface of the pattern element and the first lip and the second lip that is less than the unconstrained caliper, $H_s$, of the substrate, wherein a sum of the distance, $H_p$, and distance, $H_g$, is greater than the unconstrained caliper, $H_s$, of the substrate. Thus, as the substrate carrier advances the second surface of the substrate past the slot opening, the pattern element is advanced such that the pattern surface repeatedly advances past the first lip, the slot opening, and the second lip of the slot die applicator. As discussed below, the pattern element and/or the base surface of the substrate carrier may be compliant or compressible. And as such, the pattern element and/or the base surface of the substrate carrier is intermittently compressed as the substrate advances between the slot die applicator and the pattern surface. As such, the pattern surface of the pattern element deflects away from the slot die applicator as the substrate and the pattern element advance past the first lip, the slot opening, and the second lip of the slot die applicator. As the pattern surface is intermittently deflected away from the slot die applicator, adhesive discharged from the slot die applicator is applied onto the second surface of the advancing substrate. More particularly, the adhesive is applied to the substrate in an area having a shape that is substantially the same as a shape defined by the pattern surface.

The apparatuses and methods disclosed herein may include substrate carriers having various configurations. For example, in some embodiments the substrate carrier may be configured as a roller. In other embodiments, the substrate carrier may include an endless belt. The substrate carriers may also utilize various outer surface arrangements. For example, the base surface may be configured as a continuous surface and the substrate carrier may include a plurality of discrete pattern elements separated from each other by the continuous surface. In such a configuration, each pattern element may include a pattern surface and each pattern element may protrude outward from the continuous surface such that each pattern surface is separated from the continuous surface by the distance, $H_p$. In another example, the pattern surface may be configured as a continuous surface and the base surface may include a plurality of discrete base surfaces separated from each other by the pattern element. In such a configuration, the pattern element may protrude outward from each of the base surfaces such that each base surface is separated from the continuous surface by the distance, $H_p$. It is to be appreciated that the pattern surface of the pattern element may be configured in various different shapes and sizes and may be configured to define various different patterns. As such, adhesive may be transferred from the slot die applicator to define various patterns on a substrate.

As mentioned above, apparatuses and methods of the present disclosure may be utilized to apply adhesives to continuous substrates used in the manufacture of absorbent articles. Such substrates may be utilized in absorbent article components such as, for example: backsheets, topsheets, absorbent cores, front and/or back ears, fastener components, and various types of elastic webs and components such as leg elastics, barrier leg cuff elastics, and waist elastics. Exemplary descriptions of absorbent article components and substrates are provided below with reference to Figure 11. In addition, substrates may include continuous webs of material and component parts mounted on carrier substrates or may be in the form of a continuous substrate.

Although much of the present disclosure is provided in the context of manufacturing absorbent articles, it is to be appreciated that the apparatuses and methods disclosed herein may be applied to the manufacture of other types of articles and products manufactured from continuous substrates. Examples of other products include absorbent articles for inanimate surfaces such as consumer products whose primary function is to absorb and retain soils and wastes that may be solid or liquid and which are removed from inanimate surfaces such as floors, objects, furniture and the like. Non-limiting examples of absorbent articles for inanimate surfaces include dusting sheets, pre-moistened wipes or pads, pre-moistened cloths, paper towels, dryer sheets and dry-cleaning clothes such. Additional examples of products include absorbent articles for animate surfaces whose primary function is to absorb and contain body exudates and, more specifically, devices which are placed against or in proximity to the body of the user to absorb and contain the various exudates discharged from the body. Non-limiting examples of incontinent absorbent articles include diapers, training and pull-on pants, adult incontinence briefs and undergarments, feminine hygiene garments such as panty liners, absorbent inserts, and the like, toilet paper, tissue paper, facial wipes or clothes, and toilet training wipes. Still other examples of products may include packaging components and substrates and/or containers for laundry detergent and coffee, which may be produced in pellets or pouches and may be manufactured in a converting or web process or even discreet products produced at high speed such as high-speed bottling lines, cosmetics, razor blade cartridges, and disposable consumer batteries.

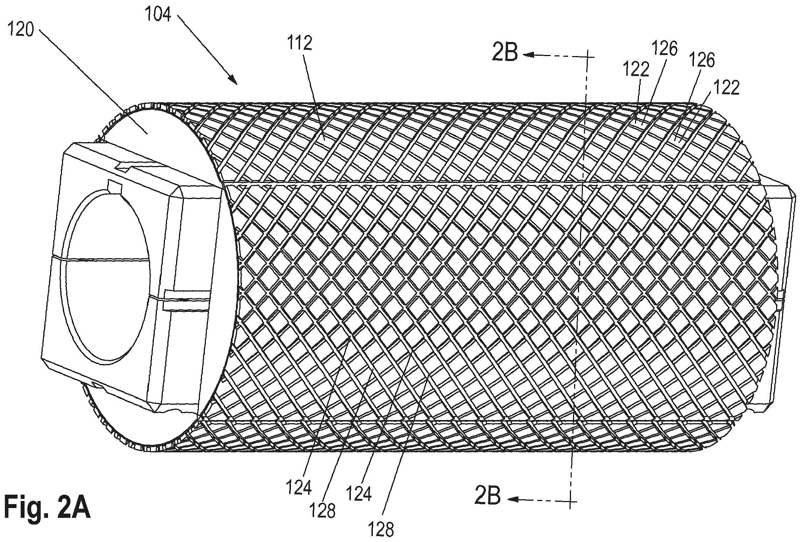

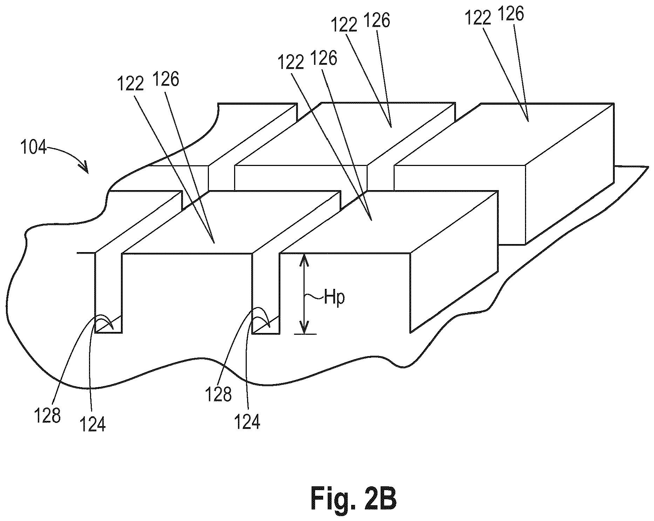

Various types of substrate carriers 104 may be used in accordance with the apparatuses and methods herein. For example, Figures 2A and 2B show an embodiment of a substrate carrier 104 configured as a roller 120 adapted to advance a substrate 106 past the slot die applicator 102. The outer surface 112 of the substrate carrier 104 shown in Figures 2A and 2B includes a plurality of pattern elements 122 that protrude radially outward from a base surface 124. Each pattern element 122 includes a pattern surface 126, and the radial protrusion of the pattern elements 122 from the base surface 124 define a distance, $H_p$, between the pattern surface 126 and the base surface 124. As shown in Figure 2A and 2B, the base surface 124 is configured as a continuous surface 128, and the plurality of discrete pattern elements 122 are separated from each other by the continuous surface 128. The pattern surfaces 126 in Figures 2A and 2B define a diamond shape. In some embodiments, the shape and size of the pattern surface 126 of each pattern element 122 may be identical or substantially identical to each other. It is to be appreciated that the number, size, and shape of some or all the pattern surfaces and/or pattern elements may be different. In addition, the distance, $H_p$, between the base surface 124 and the pattern surface 126 of the pattern element 122 may be the same or different for some or all of the pattern elements.

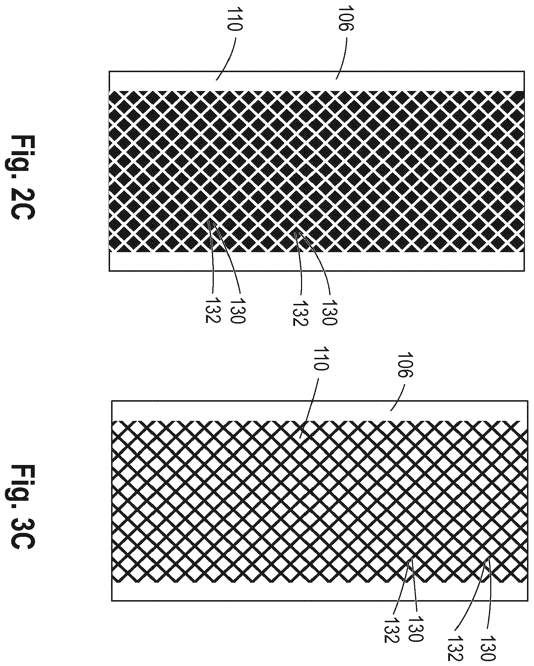

As discussed in more detail below, as the substrate carrier 104 advances the substrate 106 past the slot die applicator 102, fluid discharged from the slot die applicator is deposited onto the substrate in a pattern substantially matching the shapes of the pattern surfaces on the substrate carrier. For example, Figure 2C shows an example pattern of fluid 130 deposited on a second surface 110 of a substrate 106 after being advanced past a slot die applicator while disposed on a substrate carrier having pattern elements 122 and pattern surfaces 126 similar to those shown in Figures 2A and 2B. As shown in Figure 2C, the fluid 130 is deposited onto the substrate 106 in discrete pattern areas 132 having diamond shapes that correspond with and may mirror the shapes of the pattern surfaces 126 on the substrate carrier 104 shown in Figure 2A.

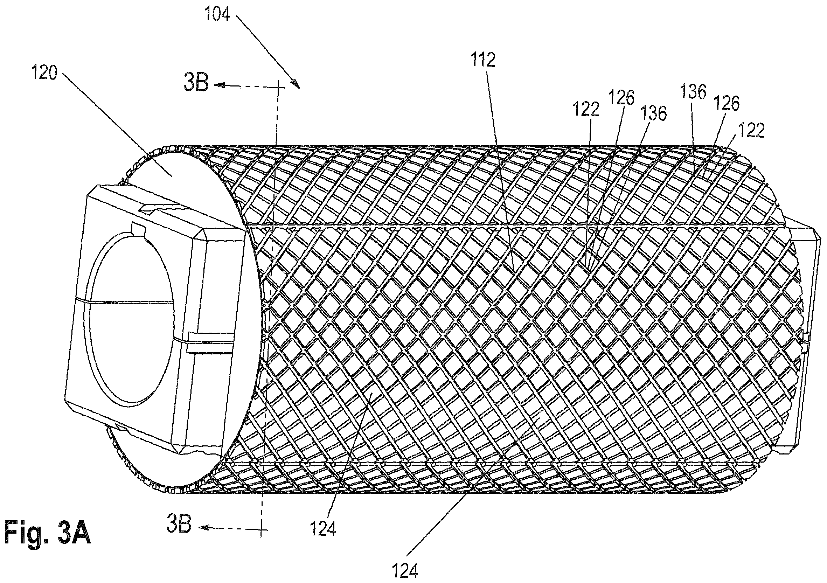

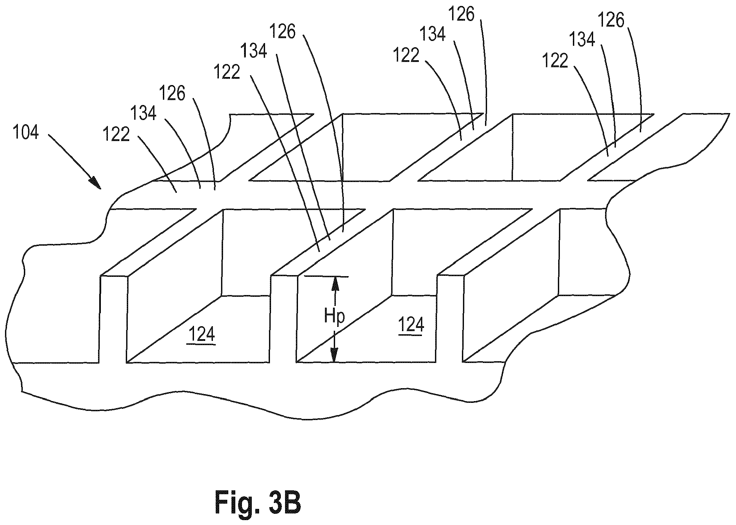

Figures 3A and 3B show another embodiment of a substrate carrier 104 configured as a roller 120 adapted to advance a substrate 106 past the slot die applicator 102. The substrate carrier 104 shown in Figures 3A and 3B includes a single pattern element 122 including a pattern surface 126. And the pattern element 122 protrudes radially outward from a plurality of base surfaces 124. More particularly, the pattern surface 126 is configured as a continuous surface 134 and the plurality of base surfaces are separated from each other by the pattern element 122. The radial protrusion of the pattern element 122 from the base surfaces 124 defines a distance, $H_p$, between the pattern surface 126 and the base surfaces 124. The pattern surface 126 in Figures 3A and 3B defines a continuous crossing line pattern wherein the shape and size of each base surface 124 are identical or substantially identical to each other. It is to be appreciated that the number, size, and shape of some or all the base surfaces may be different. In addition, the distance, $H_p$, between the base surfaces 124 and the pattern surface 126 of the pattern element 122 may be the same or different for some or all of the base surfaces. It should also be appreciated that the substrate carrier may be configured without base surfaces. For example, the substrate carrier may include a plurality of holes and the pattern surface may be configured as a continuous surface wherein the plurality of holes are separated from each other by the pattern element.

As previously mentioned, as the substrate carrier 104 advances the substrate 106 past the slot die applicator 102, fluid 130 discharged from the slot die applicator 102 is deposited onto the substrate 106 in a pattern substantially matching the shape of the pattern surface 126 on the substrate carrier 104. For example, Figure 3C shows an example pattern of fluid 130 deposited on a second surface 110 of a substrate 106 after being advanced past a slot die applicator 102 while disposed on a substrate carrier 104 having a pattern element 122 and pattern surface 126 similar to that shown in Figures 3A and 3B. As shown in Figure 3C, the fluid 130 is deposited onto the substrate 106 in a crossing line pattern defining diamond shapes therebetween that correspond with and may mirror the shapes of the base surfaces 124 on the substrate carrier 104 shown in Figures 3A and 3B.

As previously mentioned, the substrate carrier may be constructed in various ways such that the base surface and/or pattern elements may include compliant materials. In some configurations, the compliant material(s) may be compressible to allow a pattern surface of a pattern element to deflect away from the slot die applicator. Thus, the substrate carrier may be configured such that deflection of the pattern surface away from the slot die applicator compresses the pattern element and/or base surface as the substrate and the pattern element advance past the first lip, the slot opening, and the second lip of the slot die applicator.

Figure 4 shows a schematic cross-sectional side view of an example substrate carrier 104 that may be configured with compliant materials and components that can be compressed and allow the pattern surface 126 to deflect in response to a force or forces, F, exerted on the pattern surface 126. The substrate carrier 104 in Figure 4 is in the form of a roller 120 adapted to rotate around an axis of rotation 105. In operation, a force or forces, F, may be exerted on the pattern surface 126 as the substrate 106 and the pattern element 122 advance past the first lip 116, the slot opening 114, and the second lip 118 of the slot die applicator 102. It is to be appreciated that the substrate carrier 104 may be configured in various ways with various different components of compliant materials that allow the pattern surface 126 to deflect.

For example, Figures 4A1 and 4A2 show a detailed view of the substrate carrier 104 in the form of a roller 120, such as from Figure 4, including a compliant pattern element 122 and a compliant base surface 124 connected with a base roll 160 having a non-compliant support surface 162. More particularly, the roller 120 in Figures 4A1 and 4A2 may include a base layer 164 of compliant material extending radially outward from the non-compliant support surface 162 to define the compliant base surface 124. In some arrangements, the base layer 164 of compliant material may be formed as a cylindrically shaped sleeve or tube 166 having an inner radial surface 168 and an outer radial surface 170. The inner radial surface 168 may surround all or a portion of the non-compliant support surface 162 of the base roll 160, and the outer radial surface 170 may define all or a portion of the base surface 124. In turn, the pattern element 122 may include a proximal end portion 172 and a distal end portion 174 that includes the pattern surface 126, wherein the proximal end portion 172 is connected with outer radial surface 170 of the base layer 164. As such, the pattern element 122 may extend radially outward from the base layer 164 of compliant material to the distal end portion 174. It is to be appreciated that the pattern element 122 may be separately connected with or integrally formed with the compliant base layer 164. Figure 4A1 shows the pattern element 122 and base layer 164 of compliant material in an uncompressed state, wherein the minimum distance between the pattern surface 126 and the non-compliant support surface 162 is defined by distance, R1. Figure 4A2 shows the compliant pattern element 122 and compliant base layer 164 of Figure 4A1 in a compressed state wherein a force or forces, F, are applied to the pattern surface 126. Because the pattern element 122 and base layer 164 are both compliant, the force or forces, F, applied to the pattern surface 126 causes the pattern element 122 and the base layer 164 to be compressed against the non-compliant surface 162 of the base roll 160. The compression of the pattern element 122 and the base layer 164 allows the pattern surface 126 to deflect in response to the forces, F. As such, the minimum distance between the pattern surface 126 and the non-compliant surface 162 is defined as distance, R2, wherein R2 is less than R1.

In another example, Figures 4B1 and 4B2 show a detailed view of the substrate carrier 104 in the form of a roller 120, such as from Figure 4, including a non-compliant pattern element 122 and a compliant base surface 124 connected with a base roll 160 having a non-compliant support surface 162. More particularly, the roller 120 in Figures 4B1 and 4B2 may include a base layer 164 of compliant material extending radially outward from the non-compliant support surface 162 to define the compliant base surface 124. In some arrangements, the base layer 164 of compliant material may be formed as a cylindrically shaped sleeve or tube 166 having an inner radial surface 168 and an outer radial surface 170. The inner radial surface 168 may surround all or a portion of the non-compliant support surface 162 of the base roll 160, and the outer radial surface 170 may define all or a portion of the base surface 124. In turn, the pattern element 122 may include a proximal end portion 172 and a distal end portion 174 that includes the pattern surface 126, wherein the proximal end portion 172 is connected with outer radial surface 170 of the base layer 164. As such, the pattern element 122 may extend radially outward from the base layer 164 of compliant material to the distal end portion 174. It is to be appreciated that the pattern element 122 may be separately connected with or integrally formed with the compliant base layer 164. Figure 4B1 shows the base layer 164 of compliant material in an uncompressed state, wherein the minimum distance between the pattern surface 126 and the non-compliant support surface 162 is defined by distance, R1. Figure 4B2 shows the compliant base layer 164 of Figure 4B1 in a compressed state wherein a force or forces, F, are applied to the pattern surface 126. Because the pattern element 122 is non-compliant and the base layer 164 is compliant, the force or forces, F, applied to the pattern surface 126 causes the pattern element 122 to push against the base layer 164 such that the base layer 164 is compressed between the pattern element 122 and the non-compliant surface 162 of the base roll 160. The compression of the base layer 164 allows the pattern surface 126 to deflect in response to the force or forces, F. As such, the minimum distance between the pattern surface 126 and the non-compliant surface 162 is defined as distance, R2, wherein R2 is less than R1.

In yet another example, Figures 4C1 and 4C2 show a detailed view of the substrate carrier 104 in the form of a roller 120 from Figure 4 including a compliant pattern element 122 connected with a base roll 160. The base roll 160 includes a non-compliant outer circumferential support surface 162 that also defines the base surface 124. In turn, the pattern element 122 may include a proximal end portion 172 and a distal end portion 174 that includes the pattern surface 126, wherein the proximal end portion 172 is connected with non-compliant support surface 162. Figure 4C1 shows the pattern element 122 in an uncompressed state, wherein the minimum distance between the pattern surface 126 and the non-compliant support surface 162 is defined by distance, R1. Figure 4C2 shows the pattern element 122 of Figure 4C1 in a compressed state wherein a force or forces, F, are applied to the pattern surface 126. Because the pattern element 122 is compliant, the force or forces, F, applied to the pattern surface 126 causes the pattern element 122 to be compressed against the non-compliant support surface 162 of the base roll 160. The compression of the pattern element 122 allows the pattern surface 126 to deflect in response to the force or forces, F. As such, the minimum distance between the pattern surface 126 and the non-compliant support surface 162 is defined as distance, R2, wherein R2 is less than R1. In some instances, the force or forces, F, may be exerted in a radial direction toward the axis of rotation 105.

As previously mentioned, the methods and apparatuses herein include a substrate carrier adapted to advance a substrate past a slot die applicator. Figure 5 shows a schematic cross-sectional side view of an embodiment of a fluid application apparatus 100 including a substrate carrier 104 and a slot die applicator 102. The substrate 106 includes a first surface 108 and a second surface 110 disposed opposite the first surface 108. A portion of the first surface 108 of the substrate 106 is disposed on the substrate carrier 104, which may be configured as a roller 120 having a plurality of pattern elements 122 protruding from a plurality of base surfaces 124. It is to be appreciated that the substrate carrier 104 shown in Figure 5 may be configured with various features and aspects of any substrate carriers discussed herein, including those discussed above with reference to Figures 1 through 4C2. The roller 120 rotates to advance the second surface 110 of the substrate 106 past the slot die applicator 102. A fluid delivery system 138 may be used to supply fluid 130, such as an adhesive, to the slot die applicator 102. It is to be appreciated that the fluid delivery system may be configured in various different ways. For example, as shown in Figure 5, the fluid delivery system 138 may include a pump 140 to move fluid from a tank 142 to the slot die applicator 102. The fluid delivery system 138 may also be configured with a pressure relief valve 144 configured to help control the pressure of the fluid 130 fed from the pump 140. Fluid 130 from the fluid delivery system 138 passes through the slot die applicator 102 and slot opening 114 and is transferred to the second surface 110 of the advancing substrate 106.

With continued reference to Figure 5, fluid 130 passing from the slot die applicator 102 is transferred to the second surface 110 of the substrate 106 in a pattern or shape that is substantially the same as the pattern surfaces 126 on the substrate carrier 104. As discussed in more detail below, the substrate carrier 104 is positioned adjacent the slot die applicator 102 to define a minimum distance between the pattern surface 126 and slot die applicator 102, which is less than the unconstrained caliper of the substrate 106. As such, the pattern element and/or base surface may be compressed to allow the pattern surface 126 of the pattern element to deflect away from the slot die applicator 102 as the substrate 106 and the pattern surface 126 of the pattern element 122 advances past the first lip 116, the slot opening 114, and the second lip 118 of the slot die applicator 102. However, the minimum distance between the base surface 124 of the substrate carrier 104 and the slot die applicator 102 is greater than the unconstrained caliper of the substrate 106. As such, the base surface 124 is not compressed as the substrate advances past the first lip 116, the slot opening 114, and the second lip 118 of the slot die applicator 102. Thus, in operation, although fluid 130 is continuously discharged from the slot die applicator 102, fluid 130 is transferred to the advancing substrate 106 when the pattern element 122 and/or base surface 124 is compressed as pattern surfaces 126 on the substrate carrier 102 advance past the slot die opening 114 and deflect the pattern surface 126. And fluid 130 is not transferred to the advancing substrate 106 when the pattern element 122 and/or base surface 124 are uncompressed while the base surfaces 124 on the substrate carrier 104 advance past the slot die opening 114. The following provides a more detailed description of fluid transfer from the slot die applicator to the substrate with reference to Figures 6A through 6E.

Figure 6A is a detailed cross-sectional view of the substrate carrier of Figure 5 shown without the substrate wherein the pattern surface 126 of a pattern element 122 is adjacent a first lip 116, a second lip 118, and slot opening 114 of the slot die applicator 102. As shown in Figure 6A, the substrate carrier 104 includes a non-compliant support surface 162, a base surface 124, and a pattern element 122 protruding from base surface 124. In an uncompressed state, the pattern element 122 protrudes outward from the base surface 124 to define a distance, Hp, between the pattern surface 126 and the base surface 124, and to define a minimum distance, R1, between the pattern surface 126 and the non-compliant support surface 162. The substrate carrier 104 is also positioned adjacent the slot die applicator 102 to define a minimum distance, Hg, between the pattern surface 126 of the uncompressed pattern element 122 and the first lip 116 and the second lip 118. As discussed below, the minimum distance, Hg, is less than the unconstrained caliper, Hs, of the substrate 106 advanced by the substrate carrier 104. In addition, the substrate carrier 104 is positioned adjacent the slot die applicator 102 to define a minimum distance, Hb, between the base surface 124 and the first lip

116 and the second lip 118. As discussed below, the minimum distance, Hb, may be greater than the unconstrained caliper, Hs, of the substrate advanced by the substrate carrier 104.

Figure 6B is a detailed cross-sectional view of a substrate carrier 104 of Figure 6A and a substrate 106 advancing past a slot die applicator 102. The substrate 106 has an unconstrained caliper, Hs, and has a first surface 108 disposed opposite of a second surface 110. The first surface 108 of the substrate 106 is disposed on the substrate carrier 104. And the substrate 106 and substrate carrier 104 are shown as advancing together in a machine direction, MD, past the slot die applicator 102. More particularly, the second surface 110 of the substrate 106 is advancing past a slot opening 114 located between an upstream lip 116 and a downstream lip 118 of the slot die applicator 102. As previously mentioned, the substrate carrier 104 is positioned adjacent the slot die applicator 102 to define a minimum distance, Hg, between the uncompressed pattern surface 126 of the pattern element 122 and the first lip 116 and the second lip 118 that is less than the unconstrained caliper, Hs, of the substrate 106. In addition, the substrate carrier 104 is positioned adjacent the slot die applicator 102 to define a minimum distance, Hb, between the base surface 124 and the first lip 116 and the second lip 118 that is greater than the unconstrained caliper, Hs, of the substrate. The apparatus 100 may also be configured such that a sum of the distance, Hp, and distance, Hg, is greater than the unconstrained caliper, Hs, of the substrate 106. Thus, a portion 106a of the substrate 106 that is located between the slot opening 114 of the slot die applicator 102 and the advancing base surface 124 is not pressed against the base surface 124. As such, although fluid 130 is continuously discharged from the slot opening 114, fluid 130 is not being transferred to the second surface 110 of the substrate 106.

Figure 6C is a detailed cross-sectional view of the substrate carrier 104 and substrate 106 of Figure 6B wherein the base surface 124 has advanced past the slot opening 114 of the slot die applicator 102 such that a portion 106b of the substrate 106 is between the first lip 116 of the slot die applicator 102 and a leading edge 146 of an advancing pattern surface 126. As previously discussed, the minimum distance, Hg, between the pattern surface 126 of the uncompressed pattern element 122 and the first lip 116 and the second lip 118 is less than the unconstrained caliper, Hs, of the substrate 106. As such, a portion 106b of substrate 106 between the pattern surface 126 and the first lip 116 is pressed against and exerts forces on the pattern surface 126. Thus, the pattern element 122 and/or base surface 124 compresses, allowing the pattern surface 126 to deflect away from the first lip 116 to define a minimum distance, R2, between the pattern surface 126 and the non-compliant support surface 162. The fluid 130 being discharged from the slot opening 114 is shown in Figure 6C as beginning to transfer to the second surface 110 of the substrate as the leading edge 146 of the pattern surface 126 and adjacent portion of the substrate 106 begin to advance past the slot opening 114.

With continued reference to Figure 6C, the compression of the pattern element 122 and/or base surface 124 allows the pattern surface 126 to deflect away from the first lip 116 to define a compressed distance, Hc, between the pattern surface 126 and the first lip 116. When the substrate 106 is made from a material, such as a film, the substrate 106 may maintain a caliper that is substantially the same as the unconstrained caliper, Hs, while advancing between the pattern surface 126 and the first lip 116. Thus, the pattern surface 126 may deflect by a distance represented by the difference of Hg and Hs, and in some instances, the distance R2, may be calculated as:

$$R2 = R1 + Hg - Hs$$

In such a scenario, the compressed distance, Hc, may also be equal to or substantially equal to the unconstrained caliper, Hs.

Still referring to Figure 6C, when the substrate 106 is made from a material, such as a nonwoven or laminate including a nonwoven layer, the substrate 106 may be compressed to a caliper that is less than the unconstrained caliper, Hs, while advancing between the pattern surface 126 and the first lip 116. In such a scenario, the compressed distance, Hc, may be less than the unconstrained caliper, Hs. In other words, the substrate 106 may be compressed to a caliper equal to or substantially equal the compressed distance, Hc. Thus, the pattern surface 126 may deflect by a distance represented by the difference of Hg and Hc, and in some instances, the distance R2, may be calculated as:

$$R2 = R1 + Hg - Hc$$

Figure 6D is a detailed cross-sectional view of the substrate carrier 104 and substrate of Figure 6C wherein the base surface 124 and leading edge 146 of the pattern surface 126 has advanced past the slot opening 114 of the slot die applicator 102 such that the portion 106b of the advancing substrate 106 is between the slot opening 114 of the slot die applicator 102 and an advancing pattern surface 126. Because the minimum distance, Hg, between the pattern surface 126 of the uncompressed pattern element 122 and the first lip 116 and the second lip 118 is less than the unconstrained caliper, Hs, of the substrate 106, the portion 106b of substrate 106 between the pattern surface 126 and the first lip 116 and second lip 118 of the slot die applicator 102 presses against and exerts forces on the pattern surface 126. As such, the compliant pattern element 122 and/or base surface 124 are compressed, allowing the pattern surface 126 to deflect away from the first lip 116 and second lip 118. As mentioned above, when the substrate 106 is made from a material, such as a film, the substrate 106 may maintain a caliper that is substantially the same as the unconstrained caliper, Hs, while advancing between the pattern surface 126 and the first lip 116 and second lip 118. Thus, the pattern surface 126 may deflect by a distance represented by the difference of Hg and Hs, and in some instances, the distance R2, may be calculated as: R2 = R1+Hg-Hs. Also, as mentioned above, when the substrate 106 is made from a material, such as a nonwoven or laminate including a nonwoven layer, the substrate 106 may be compressed to a caliper that is less than the unconstrained caliper, Hs, while advancing between the pattern surface 126 and the first lip 116 and second lip 118. Thus, the pattern surface 126 may deflect by a distance represented by the difference of Hg and Hc, and in some instances, the distance R2, may be calculated as: R2 = R1+Hg-Hc. The fluid 130 being discharged from the slot opening 114 is shown in Figure 6D as being transferred to the second surface 110 of the substrate as the pattern surface 126 and adjacent portion 106b of the substrate 106 advance past the slot opening 114.

Figure 6E is a detailed cross-sectional view of the substrate carrier 104 and substrate 106 of Figure 6D wherein the portion 106b of the substrate and the pattern surface 126 have advanced past the slot opening 114 of the slot die applicator 102. As shown in Figure 6E, an upstream portion 126a of the pattern surface 126 is adjacent the second lip 118, and a downstream portion126b of the pattern surface 126 has advanced past the second lip 118. As such, the portion 106b of the advancing substrate 106 between the second lip 118 of the slot die applicator 102 and the upstream portion 126a of the advancing pattern surface 126 presses against and exerts forces on the pattern surface 126. As such, the compliant pattern element 122 and/or base surface 124 are compressed, allowing the upstream portion 126a of the pattern surface 126 to deflect away from the first lip 116 and second lip 118 to define the minimum distance, R2, between the upstream portion 126a of the pattern surface 126 and the non-compliant support surface 162.

With continued reference to Figure 6E, the downstream portion 126b of the pattern surface 126 has advanced past the second lip 118 of the slot die applicator 102, and as such, the portion 106b of the substrate 106 is no longer pressing against downstream portion 126b of the pattern surface 126, allowing the compliant pattern element 122 and/or base surface 124 to return to an uncompressed state wherein the downstream portion 126b of the pattern surface 126 deflects back away from the non-compliant surface 162 such that the minimum distance between the non-compliant surface 162 and the downstream portion 126b pattern surface 126 is the distance, R1. Once the upstream portion 126a of the pattern surface 126 has also advanced past the second lip 118, the remainder of the compliant pattern element 122 and/or base surface 124 may return to an uncompressed state wherein the both the upstream portion 126a and downstream portion 126b of the pattern surface 126 have deflected away from the non-compliant surface 162 such that the minimum distance between the non-compliant surface 162 and the pattern surface 126 is the distance, R1.

Still referring to Figure 6E, an uncompressed portion 106c of the advancing substrate 106 is between the slot opening 114 of the slot die applicator 102 and an advancing base surface 124. Because the minimum distance, Hb, between the base surface 124 and the first lip 116 and the second lip 118 that is greater than the unconstrained caliper, Hs, of the substrate, a portion 106c of substrate 106 that advances between the base surface 124, slot opening 114, and the first lip 116 of the slot die applicator 102 is uncompressed. As such, the fluid 130 being discharged from the slot opening 114 is shown in Figure 6E as ceasing to be transferred to the second surface 110 of the substrate 106 as the base surface 124 and adjacent uncompressed portion 106c of the substrate advance past the slot opening 114.

As previously mentioned, various forms and configurations of substrate carriers may be used with the presently disclosed methods and apparatuses. For example, Figure 7 shows a schematic cross-sectional side view of an embodiment of a fluid application apparatus 100 with a substrate carrier 104 including an endless pattern belt 148. The pattern belt 148 is wrapped around two rollers 150 adapted to advance pattern belt 148 and substrate past the slot die applicator 102. The pattern belt 148 may include various different combinations, shapes, and types of pattern elements 122 and base surfaces 124 and/or holes 136 as previous described. As shown in Figure 7, the slot die applicator 102 is adjacent the pattern belt 148 at a location where the pattern belt 148 is partially wrapped around one of the rollers 150. It is to be appreciated that the slot die applicator 102 may be located adjacent other locations of the pattern belt 148. For example, Figure 8 shows a schematic cross-sectional side view of an embodiment of a fluid application apparatus 100 wherein the slot die applicator 102 is adjacent the pattern belt 148 at a location between the rollers 150. And Figure 9 shows a schematic cross-sectional side view of the embodiment of Figure 8 with a backup plate 152 located behind the pattern belt 148, wherein the backup plate 148 provides support to the pattern belt 148 to help prevent the pattern belt from deflecting away from the slot die applicator 102.

With reference to the above description and associated figures, it is to be appreciated that the apparatuses 100 herein may be used to apply adhesive 130 discharged from a slot die applicator 102 to a substrate 106 in a pattern by continuously advancing the substrate in a machine direction past a first lip 116, second lip 118, and slot opening 114 in the slot die applicator 102. The substrate 106 may be engaged with a substrate carrier 104 that may include a base surface 124 and a pattern element 122, wherein the pattern element includes a pattern surface 126. The pattern element 122 protrudes from the base surface 124 to define a distance, Hp, between the pattern surface 126 and the base surface 124. As previously mentioned, in some embodiments, the substrate carrier may include holes 136 instead of or in combination with base surfaces 126 adjacent the pattern element 122. The substrate carrier 104 is positioned adjacent the slot die applicator 102 to define a minimum distance, Hg, between the pattern surface 126 of the uncompressed pattern element 122 and the first lip 116 and the second lip 118 that is less than the unconstrained caliper, Hs, of the substrate 106. The second surface 110 of the substrate 106 may be advanced past the slot die applicator 102 while the first surface 108 of the substrate 106 is disposed on the substrate carrier 104. And the substrate 106 is intermittently compressed between the slot die applicator 102 and the pattern surface 126 of the pattern element 122 by advancing the pattern element as the pattern surface of the pattern element advances past the first lip 116, the slot opening 114, and the second lip 118 of the slot die applicator 102 while the first surface 108 of the substrate 106 is disposed on the substrate carrier 104.

It is to be appreciated that the methods and apparatuses herein may deposit fluids, such as adhesives, onto advancing a substrate advancing in a machine direction MD in various designs or patterns. For example, Figure 10A shows fluid 130 deposited onto the second surface 110 of a substrate 106 in an example pattern defined by discrete pattern areas 132 having varying cross directional CD widths and/or cross directional CD locations. In addition, because the fluid 130 is deposited onto the substrate 106 in pattern areas 132 having shapes that correspond with and may mirror the shapes of the pattern surfaces 126 of the pattern elements 122 as discussed above, the fluid 130 may be deposited intermittently to define distances, dp, between pattern areas 132 along the machine direction MD that correspond with the distances between adjacent pattern surfaces 126 on the substrate carrier 104. In some configurations, the fluid 130 may be deposited onto the substrate intermittently to define distances between pattern areas 132 of 30 mm or less along the machine direction of the substrate 106. In addition, the fluid 130 may be deposited on the substrate 106 so as to create a varying thickness that defines a cross-sectional profile along the machine direction MD. For example, Figure 10B shows a cross-sectional view of the pattern areas 132 on the substrate 106 of Figure 10A. As shown in Figure 10B along the machine direction MD, each pattern area 132 includes a leading end portion 400 and a trailing end portion 402 separated by a central portion 404. The leading end portion 400 defines a first thickness, $t_1$, the central portion defines 404 a second thickness, $t_2$, and the trailing end portion 402 define a third thickness, $t_3$. In some configurations, the first thickness, $t_1$, is greater than the second thickness $t_2$, and the third thickness, $t_3$, and the second thickness, $t_2$, may be substantially the same as the third thickness, $t_3$.

Description of an exemplary absorbent article (diaper 250)

As previously mentioned, the apparatuses 100 and methods herein may be used to provide for the application of adhesives in patterns to substrates and components during the manufacture of various different products. The method of the invention may be advantageously used to make absorbent articles for disposable personal hygiene such as diapers. For the purposes of a specific illustration, Figure 11 shows one example of a disposable absorbent article 250, such as described in US2008/0132865A1, in the form of a diaper 250 that may be constructed from such substrates and components manipulated during manufacture according to the apparatuses and methods disclosed herein. In particular, Figure 11 is a plan view of one embodiment of a diaper including a chassis 254 shown in a flat, unfolded condition, with the portion of the diaper that faces a wearer oriented towards the viewer. A portion of the chassis structure is cut-away in Figure 11 to more clearly show the construction of and various features that may be included in embodiments of the diaper.

As shown in Figure 11, the diaper 250 includes a chassis 254 having a first ear 256, a second ear 258, a third ear 260, and a fourth ear 262. To provide a frame of reference for the present discussion, the chassis is shown with a longitudinal axis 264 and a lateral axis 266. The chassis 254 is shown as having a first waist region 268, a second waist region 270, and a crotch region 272 disposed intermediate the first and second waist regions. The periphery of the diaper is defined by a pair of longitudinally extending side edges 274, 276; a first outer edge 278 extending laterally adjacent the first waist region 268; and a second outer edge 280 extending laterally adjacent the second waist region 270. As shown in Figure 11, the chassis 254 includes an inner, body-facing surface 282, and an outer, garment-facing surface 284. A portion of the chassis structure is cut-away in Figure 11 to more clearly show the construction of and various features that may be included in the diaper. As shown in Figure 11, the chassis 254 of the diaper 250 may include an outer covering layer 286 including a topsheet 288 and a backsheet 290. An absorbent core 292 may be disposed between a portion of the topsheet 288 and the backsheet 290. As discussed in more detail below, any one or more of the regions may be stretchable and may include an elastomeric material or laminate as described herein. As such, the diaper 250 may be configured to adapt to a specific wearer's anatomy upon application and to maintain coordination with the wearer's anatomy during wear.

The absorbent article may also include an elastic waist feature 202 shown in Figure 11 in the form of a waist band 294 and may provide improved fit and waste containment. The elastic waist feature 202 may be configured to elastically expand and contract to dynamically fit the wearer's waist. The elastic waist feature 202 can be incorporated into the diaper in accordance with the methods discussed herein and may extend at least longitudinally outwardly from the absorbent core 292 and generally form at least a portion of the first and/or second outer edges 278, 280 of the diaper 252. In addition, the elastic waist feature may extend laterally to include the ears. While the elastic waist feature 202 or any constituent elements thereof may comprise one or more separate elements affixed to the diaper, the elastic waist feature may be constructed as an extension of other elements of the diaper, such as the backsheet 290, the topsheet 288, or both the backsheet and the topsheet. In addition, the elastic waist feature 202 may be disposed on the outer, garment-facing surface 284 of the chassis 240; the inner, body-facing surface 282; or between the inner and outer facing surfaces. The elastic waist feature 202 may be constructed in a number of different configurations including those described in U.S. Patent No. 7,432,413; U.S. Patent Publication No. 2007/0142798; and US2007/0287983.

As shown in Figure 11, the diaper 252 may include leg cuffs 296 that may provide improved containment of liquids and other body exudates. In particular, elastic gasketing leg cuffs can provide a sealing effect around the wearer's thighs to prevent leakage. It is to be appreciated that when the diaper is worn, the leg cuffs may be placed in contact with the wearer's thighs, and the extent of that contact and contact pressure may be determined in part by the orientation of diaper on the body of the wearer. The leg cuffs 296 may be disposed in various ways on the diaper 202.

The diaper 252 may be provided in the form of a pant-type diaper or may alternatively be provided with a re-closable fastening system, which may include fastener elements in various locations to help secure the diaper in position on the wearer. For example, fastener elements may be located on the first and second ears and may be adapted to releasably connect with one or more corresponding fastening elements located in the second waist region. It is to be appreciated that various types of fastening elements may be used with the diaper.

Components of the disposable absorbent article (i.e., diaper, disposable pant, adult incontinence article, sanitary napkin, pantiliner, etc.) described in this specification can at least partially be comprised of bio-sourced content as described in US 2007/0219521A1 Hird et al published on September 20, 2007, US 2011/0139658A1 Hird et al published on June 16, 2011, US 2011/0139657A1 Hird et al published on June 16, 2011, US 2011/0152812A1 Hird et al published on June 23, 2011, US 2011/0139662A1 Hird et al published on June 16, 2011, and US 2011/0139659A1 Hird et al published on June 16, 2011. These components include, but are not limited to, topsheet nonwovens, backsheet films, backsheet nonwovens, side panel nonwovens, barrier leg cuff nonwovens, super absorbent, nonwoven acquisition layers, core wrap nonwovens, adhesives, fastener hooks, and fastener landing zone nonwovens and film bases.

In at least one exemplary configuration, a disposable absorbent article component comprises a bio-based content value from about 10% to about 100% using ASTM D6866-10, method B, in another embodiment, from about 25% to about 75%, and in yet another embodiment, from about 50% to about 60% using ASTM D6866-10, method B.

In order to apply the methodology of ASTM D6866-10 to determine the bio-based content of any disposable absorbent article component, a representative sample of the disposable absorbent article component must be obtained for testing. In at least one embodiment, the disposable absorbent article component can be ground into particulates less than about 20 mesh using known grinding methods (e.g., Wiley® mill), and a representative sample of suitable mass taken from the randomly mixed particles.

In the context of the previous discussion, the apparatuses 100 and methods herein may be used to provide for the application adhesives in patterns to substrates and components during the manufacture of an absorbent article. For example, adhesives may be applied in various patterns to portions of any of the topsheet, backsheet films, backsheet nonwovens, absorbent core, core encapsulation webs, acquisition layer, surge layer, secondary topsheet layer, leg cuffs, waist feature, ears, and fastening elements during the manufacture of an absorbent article. In some instances, the adhesive may be a different color than that of the substrate.

In some applications, the apparatuses and methods herein may be adapted to apply adhesives in absorbent core assembly processes, such as described for example in USUS2006/0021695A1; US2006/0048880A1; US2008/0215166A1; and US2010/0051166A1. An absorbent core typically comprises of a core wrap comprising a top side and bottom side, with the absorbent material disposed between the top side of the bottom side of the core wrap. The core wrap is typically a nonwoven. The core wrap typically comprises at least one longitudinal adhesive seal to ensure sufficient containment of the absorbent material during usage. While core wrap end seals at the front and back of the absorbent core are optional, the present invention may be particularly used to deposit at least one cross-direction oriented stripe of the polymeric onto the inner surface of the top or bottom side of the core wrap in order to make one or both of these core end wrap seal(s).

In some instances, the apparatuses and methods herein may be configured to apply fluid formulations in the form of wetness indicators, such as disclosed for example in US2011/0137274A1. In yet other instances, the apparatuses and methods herein may be configured to apply fastening adhesives for feminine care articles, including sanitary napkins, panty liners, adult incontinence pads, and the like, such as disclosed for example in EP0745368A1.

Application pattern

While the invention can be used to make conventional application patterns such as stripes oriented in machine-direction, the invention also enables application patterns with characteristics which are not accessible via standard slot coating process. Thus, in another aspect, the invention is for an absorbent article comprising a substrate with a polymeric composition as described above applied thereon via a contact applicator, but which does not consist solely of one or more slots oriented in the machine direction as is known from conventional slot applicators.

The application pattern may in particular comprise a pattern element having a dimension in machine direction of less than 10 mm. The pattern element may be continuous, for example with the composition applied forming a grid pattern as illustrated in Fig. 3c, or the application pattern may comprise discrete elements such as series of geometric figures such as square, rectangular, circular, oval or triangular shape, e.g. applied in a regular or irregular pre-determined pattern, as illustrated in Fig. 2c. The invention allows pattern elements having at their shortest extent in MD and/or in CD a dimension of down to 0.3 mm. The discrete pattern elements may be separated by a gap of down to 0.1 mm in MD and down to 0.1 mm in CD. Discrete elements having asymmetric shapes like artwork, letters, curved lines, logo etc... may be obtained with the method of the invention when using a patterned substrate carrier.

The invention can also be used to deposit the polymeric composition in the form of one or more CD oriented stripes. This type of application is especially relevant for sealing applications between the substrates, for example the front and back core wrap seals.

Polymeric composition

The term "polymeric composition" refers to a composition comprising at least 50 % by weight of one or more polymers, by weight of the polymeric composition. The present invention is applicable such polymeric compositions, which may typically comprise higher amount, such as at least 75%, or at least 80%, or at least 85%, or at least 90%, or at least 95% and in some case up to 100% or up to 98% of one or more polymers by weight of the composition. Blend of two or more polymers may be used, but one advantage of the invention is that it allows application of a polymeric composition that does not require formulations of multiple polymers. In the following, the term "polymer" is used to indicate "one or more polymer". The polymer may be a copolymer or a homopolymer. The polymer used in the polymeric composition may in particular comprise one or more an amorphous poly alpha olefin.

The polymeric compositions of the invention have a Tensile Strength at Yield of from about 0.5 MPa to about 10 MPa, as measured as indicated in the ASTM method below. As indicated previously, it was found that these polymeric compositions are typically difficult to apply using conventional slot coating technology without blobbing. The polymeric composition used in the present invention may in particular have a Tensile Strength at Yield of from about 1 MPa to about 8 MPa, alternatively from about 2 MPa to about 6 MPa, alternatively from about 4 MPa to about 7 MPa, alternatively from about 4.5 MPa to about 6 MPa, alternatively from about 4.94 MPa to about 5.76 MPa, alternatively from about 0.5 MPa to about 4.5 MPa, alternatively from about 1 MPa to about 4 MPa, alternatively from about 2 MPa to about 4 MPa, alternatively from about 2.06 MPa to about 3.53 MPa, and alternatively from about 0.5 MPa to about 7 MPa, according to the Tensile Strength Test Method described herein.

The polymer of the invention may advantageously comprise or consists of a propylene-based polymer, in particular a propylene-butene copolymer, a polypropylene homopolymer, a propylene ethylene copolymer or any mixtures thereof.

The polymer can comprise 100%, alternatively from about 75% to about 95%, alternatively from about 80% to about 90%, alternatively from about 30 % to about 70 %, alternatively from about 35 % to about 65 %, alternatively from about 40 % to about 60 %, alternatively from about 45 % to about 55 %, alternatively from about 10 % to about 20 %, alternatively from about 15 % to about 25 %, alternatively from about 1% to about 5%, and alternatively from about 5% to about 12 % of propene monomer units, by weight of the polymer. The percentage of propene monomer units may be determined by a suitable method, such as nuclear magnetic resonance or infrared spectroscopies, known to those of skill in the art.

The polymer can comprise 100%, alternatively from about 75% to about 95%, alternatively from about 80% to about 90%, alternatively from about 30 % to about 70 %, alternatively from about 35 % to about 65 %, alternatively from about 40 % to about 60 %, alternatively from about 45 % to about 55 %, alternatively from about 10 % to about 20 %, and alternatively from about 15 % to about 25 %, alternatively from about 1% to about 5%, and alternatively from about 5% to about 12 % of ethylene monomer units, by weight of the polymer. The percentage of ethylene monomer units may be determined by a suitable method, such as nuclear magnetic resonance or infrared spectroscopies, known to those of skill in the art.

The polymer can comprise 100%, alternatively from about 75% to about 95%, alternatively from about 80% to about 90%, alternatively from about 30 % to about 70 %, alternatively from about 35 % to about 65 %, alternatively from about 40 % to about 60 %, alternatively from about 45 % to about 55 %, alternatively from about 10 % to about 20 %, and alternatively from about 15 % to about 25 %, alternatively from about 1% to about 5%, and alternatively from about 5% to about 12 % of 1-butene monomer units, by weight of the polymer. The percentage of 1-butene monomer units may be determined by a suitable method, such as nuclear magnetic resonance or infrared spectroscopies, known to those of skill in the art.

If the polymer is a propylene based copolymer, in particular a propylene-ethylene copolymer, it can also comprise from about 1 % to about 40 %, alternatively from about 2 % to about 30 %, alternatively from about 5 % to about 20 %, and alternatively from about 10 % to about 15 %, by weight of the copolymer, of one or more comonomer units selected from the group consisting of 4-methyl-1-pentene, pentene-1, 2-methylpentene-1, 3-methylbutene-1, heptene-1, dimethylpentene-1, trimethylbutene-1, ethylpentene-1, methylpentene-1, trimethylpentene-1, methylethylpentene-1, 1-octene, diethylbutene-1, propylpentane-1, decene-1, methylnonene-1, nonene-1, trimethylheptene-1, methylethylbutene-1, dodecene-1, and hexadodecene-1, and combinations thereof.

The polymer can be prepared by the methods described in US5,302,675 and 5,723,546. The polymer may be prepared using a single-site catalyst system, multiple single-site catalyst systems, or Ziegler Natta catalyst system. Monomers used to prepare the copolymer can be obtained from one or more carbon-based sources, e.g., biomass from animal and/or vegetable fats. The monomers can also be obtained from renewable feed stocks provided by, e.g., Neste's Rotterdam Refinery (Neste, Finland). Polymeric compositions comprising a polymer can be prepared by combining the polymer and at least one optional ingredient (e.g., an optical brightener, other copolymers), if desired. The polymer can be prepared into a final polymeric composition by heating the primary polymer to elevated temperatures (e.g., about 135 to about 175° C) that melts the polymer. Once molten, one or more optional ingredients (e.g., additive or other polymers components) can be added to the primary polymer. A mixer can be used to mix the components together into a final polymeric composition.

The polymeric composition may comprise a homopolymer of propylene. The homopolymer or propylene may be made using one or more metallocene catalysts. The homopolymer of propylene may have low modulus compared to typical homopolymers of propylene due to less stereoregularity. The homopolymer or propylene may comprise 0% tackifier. The homopolymer or propylene may have a number average molecular weight of from about 20,000 g/mole to about 70,000 g/mole, alternatively from about 30,000 g/mole to about 60,000 g/mole, alternatively from about 40,000 g/mole to about 50,000 g/mole, and alternatively from about 43,000 g/mole to about 47,000 g/mole. The homopolymer or propylene may have a viscosity of from about 5,000 mPa·s to about 12,000 mPa s, alternatively from about 7,000 mPa s to about 10,000 mPa s, alternatively from about 8,000 mPa·s to about 9,000 mPa s, and alternatively about 8,500 mPa·s at 190°C according to ASTM D3236-15. An example of a homopolymer or propylene may be L-Modu S410 from Idemitsu.

The polymeric composition may comprise an amorphous poly-alphaolefin. The amorphous poly alpha olefin may be a copolymer comprising propylene and 1-butene. The amorphous poly alpha olefin may be produced using a Ziegler-Natta catalyst. The amorphous poly alpha olefin may comprise 0% tackifier. The amorphous poly alpha olefin may have a viscosity of about 3,000 mPa s, alternatively from about 2,000 mPa·s to about 5,000 mPa s, and alternatively from about 2,500 mPa·s to about 4,500 mPa·s at 190° C according to ASTM D3236-15. An example of an amorphous polyalpha olefin may be RT2830 from REXtac.

The polymeric composition may comprise a copolymer comprising propylene and ethylene. The copolymer comprising propylene and ethylene may be made using one or more metallocene catalysts. The copolymer comprising propylene and ethylene may comprise 0% tackifier. The copolymer comprising propylene and ethylene may have a viscosity of about 3,000 mPa s, alternatively from about 1,000 mPa·s to about 5,000 mPa s, alternatively from about 1,500 mPa·s to about 4,000 mPa s, alternatively from about 1,750 mPa·s to about 3,000 mPa s, alternatively from about 2,000 mPa·s to about 2,500 mPa s, alternatively from about 2,000 mPa·s to about 2,200 mPa s, and alternatively about 2,100 mPa·s at 170° C according to ASTM D3236-15. An example of a copolymer comprising propylene and ethylene may be Licocene 2502 from Clariant.

The polymeric composition may comprise a homopolymer of propylene. The homopolymer of propylene may comprise 0% tackifier. The homopolymer of propylene may have a viscosity of from about 500 mPa s to about 3,000 mPa.s, alternatively from about 750 mPa s to about 2,250 mPa s, and alternatively from about 1,200 mPa s to about 1,800 mPa s at 170° C according to ASTM D3236-15. The homopolymer of propylene may be made using one or more metallocene catalysts. An example of a homopolymer of propylene may be Licocene 6502 from Clariant.

The polymeric composition can be advantageously substantially free of tackifier. The polymeric composition can thus comprise less than 5 %, alternatively less than 3 %, alternatively less than 2 %, alternatively less than 1 %, alternatively less than 0.5 %, and alternatively less than 0.1 % of a tackifier, by weight of the polymer composition. Exemplary tackifiers can include aliphatic hydrocarbon resins, aromatic modified aliphatic hydrocarbon resins, hydrogenated poly-cyclopentadiene resins, poly-cyclopentadiene resins, gum rosins, gum rosin esters, wood rosins, wood rosin esters, tall oil rosins, tall oil rosin esters, poly-terpenes, aromatic modified poly-terpenes, terpene-phenolics, aromatic modified hydrogenated poly-cyclopentadiene resins, hydrogenated aliphatic resins, hydrogenated aliphatic aromatic resins, hydrogenated terpenes and modified terpenes, and hydrogenated rosin esters.

The polymeric composition may comprise 0% tackifier. There are significant advantages to minimizing or avoiding the use of a tackifier as it may reduce the cost of the polymeric composition, as well as eliminate an additional ingredient and potential issues that may be associated with supplying the additional ingredient. Furthermore, tackifiers may impart undesirable odor in disposable articles and can also act as carriers of low molecular weight plasticizers (e.g., process oils that are used in SBC based adhesives) that may weaken the polyethylene back sheet materials used in absorbent articles and textile articles.

A comparative tackified adhesive composition may be a styrenic block copolymer based adhesive comprising at least 20 % of a tackifier, by weight of the tackified adhesive composition. The adhesive may have a viscosity of from about 2,000 mPa·s to about 7,000 mPa s, alternatively from about 3,000 mPa·s to about 6,000 mPa s, alternatively from about 4,000 mPa·s to about 5,500 mPa s, and alternatively about 4,725 mPa·s at 160° C according to ASTM D3236-15. An example of this adhesive may be H2401 from Bostik.

Another comparative tackified adhesive composition may be a polyolefin based adhesive comprising at least 20 % of a tackifier, by weight of the tackified adhesive composition. The adhesive may have a viscosity of from about 3,000 mPa·s to about 9,000 mPa s, alternatively from about 4,000 mPa·s to about 7,000 mPa s, alternatively from about 5,000 mPa·s to about 6,500 mPa s, alternatively from about 5,750 mPa·s to about 6,250 mPa s, and alternatively about 6,000 mPa·s at 160° C according to ASTM D3236-15. An example of this adhesive may be DM3800 from Henkel.

The polymeric composition may optionally comprise an antioxidant or a stabilizer. Any antioxidant known to a person of ordinary skill in the art may be used in the adhesion composition. Non-limiting examples of suitable antioxidants include amine-based antioxidants such as alkyl diphenyl amines, phenyl-naphthylamine, alkyl or aralkyl substituted phenyl-naphthylamine, alkylated p-phenylene diamines, tetramethyl-diaminodiphenylamine and the like; and hindered phenol compounds such as 2,6-di-t-butyl-4-methylphenol; 1,3,5-trimethyl-2,4,6-tris(3',5'-di-t-butyl-4'-hydroxybenzyl)benzene; tetra kis [(methylene(3,5-di-t-butyl-4-hydroxyhydrocinnamate)]methane (e.g., IRGANOX™ 1010, from Ciba Geigy, New York); octadecyl-3,5-di-t-butyl-4-hydroxycinnamate (e.g., IRGANOX™ 1076, commercially available from Ciba Geigy) and combinations thereof. When used, the amount of the antioxidant and/or the stabilizer in the polymeric composition can be less than 1 %, alternatively from about 0.05 % to about 0.75 %, and alternatively from about 0.1 % to about 0.5 %, by weight of the polymeric composition.

The polymeric composition may optionally comprise a UV stabilizer that may prevent or reduce the degradation of the composition by radiation. Any UV stabilizer known to a person of ordinary skill in the art may be used in the polymeric composition. Non-limiting examples of suitable UV stabilizers include benzophenones, benzotriazoles, aryl esters, oxanilides, acrylic esters, formamidine carbon black, hindered amines, nickel quenchers, hindered amines, phenolic antioxidants, metallic salts, zinc compounds, and combinations thereof. Where used, the amount of the UV stabilizer in the polymeric composition can be less than 1 %, alternatively from about 0.05 % to about 0.75 %, and alternatively from about 0.1 % to about 0.5 %, by weight of the polymeric composition

The polymeric composition may optionally comprise a brightener, colorant, and/or pigment. Any colorant or pigment known to a person of ordinary skill in the art may be used in the polymeric composition. Non-limiting examples of suitable brighteners, colorants, and/or pigments include fluorescent materials and pigments such as triazine-stilbene, coumarin, imidazole, diazole, titanium dioxide and carbon black, phthalocyanine pigments, and other organic pigments such as IRGAZINB, CROMOPHTALB, MONASTRALB, CINQUASIAB, IRGALITEB, ORASOLB, all of which are available from Ciba Specialty Chemicals, Tarrytown, N.Y. Where used, the amount of the brightener, colorant, and/or pigment in the polymeric composition can be less than 10 %, alternatively from about 0.01 % to about 5 %, and alternatively from about 0.1 % to about 2 %, by weight of the polymeric composition.

The polymeric composition may optionally comprise a fragrance such as a perfume or other odorant. Such fragrances may be retained by a liner or contained in release agents such as microcapsules that may, for example, release fragrance upon removal of a release liner from or compression on the adhesive composition. Where used, the amount of the fragrance in the polymeric composition can be less than 3 %, alternatively less than 2 %, alternatively less than 1 %, alternatively from about 0.05 % to about 0.75 %, and alternatively from about 0.1 % to about 0.5 %, by weight of the polymeric composition

The polymeric composition may have a Modulus of Elasticity of from about 40 MPa to about 500 MPa, alternatively from about 50 MPa to about 300 MPa, alternatively from about 50 MPa to about 250 MPa, alternatively from about 52.1 MPa to about 235 MPa, alternatively from about 5 MPa to about 45 MPa, alternatively from about 10 MPa to about 30 MPa, alternatively from about 15 MPa to about 25 MPa, alternatively from about 19.4 MPa to about 20.6 MPa, and alternatively from about 5 MPa to about 400 MPa according to the Modulus of Elasticity Test Method described herein.

The bond area may comprise from about 13 gsm to about 30 gsm, alternatively from about 15 gsm to about 25 gsm, alternatively from about 7 gsm to about 13 gsm, alternatively from about 9 gsm to about 11 gsm, alternatively from about 5 gsm to about 30 gsm, alternatively from about 6 gsm to about 27 gsm, alternatively from about 10 gsm to about 25 gsm, alternatively from about 20 gsm to about 30 gsm, alternatively from about 23 gsm to about 27 gsm, alternatively about 10 gsm, alternatively about 15 gsm, and alternatively about 25 gsm of the polymeric composition disposed within the bond area.

Further advantageous properties

The polymeric composition may have other advantageous properties, in particular a Toughness of at least 25

MJ.m$^{-3}$, wherein Toughness is measured according to the Extensional Test Method described herein. The Toughness of the polymeric composition may be at least 27 MJm$^{-3}$. The Toughness may also be up to 200 MJm$^{-3}$, or up to 150 MJm$^{-3}$ or up to 100 MJm$^{-3}$ or up to 60 MJm$^{-3}$. Such high Toughness values are believed to provide good bonding between two nonwovens.

The polymeric composition may also advantageously have a storage modulus (G') that is higher than $0.3 \times 10^6$ Pa at 37°C, as measured with Oscillatory Rheometry Test Method described below. The relative high G' value at 37°C is indicative of an adhesive that is not tacky during use. The hot-melt adhesive may in particular have a storage modulus (G') higher than $1.0 \times 10^7$ Pa at 37°C, or even $1.5 \times 10^7$ Pa at 37°C. On the other hand, the hot-melt adhesive may also have a storage modulus (G') that is lower than $2.0 \times 10^8$ Pa at 37°C. The hot-melt adhesive may also have a storage modulus (G') higher than $0.3 \times 10^6$ Pa at 23°C, or even higher than $1.5 \times 10^7$ Pa at 23°C, or even higher than $2.0 \times 10^7$ Pa at 23°C. On the other hand, the hot-melt adhesive may also have a storage modulus (G') that is lower than $3.0 \times 10^8$ Pa at 23°C. Typically the G' value of polymers decreases when the temperature increases.

The relatively high G' value is indicative of a polymeric composition that is not tacky in this range of temperature, which enables applications in which it can get directly into contact with the consumer, e.g. when it is not sandwiched between two substrates but only applied onto one substrate, which is exposed to the consumer.

Commercially available propylene-ethylene copolymers such as those from Clariant's Licocene have the following G', as measured as indicated below: Licocene® PP 1502 has a G' value of $1.4 \times 10^7$ Pa at 23°C and $1.0 \times 10^7$ at 37°C, Licocene® PP 1602 has a G' value of $1.2 \times 10^7$ at 23°C and $8.6 \times 10^6$ at 37°C, Licocene® PP 2502 has a G' value of $5.2 \times 10^7$ at 23°C and $3.3 \times 10^7$ at 37°C.

RT-2830 from REXtac has a G' value of $4.5 \times 10^7$ Pa at 23°C and of $2.8 \times 10^7$ Pa at 37°C, L-MODU S 410 from Idemitsu has a G' value of $1{,}4 \times 10^7$ Pa at 23°C and of $9.1 \times 10^6$ Pa at 37°C.

The hot-melt adhesive may preferably also have a storage modulus (G') higher than $0.3 \times 10^6$ at 60°C (reflecting storage conditions in countries with hot climate, as e.g. measured in containers). The hot-melt adhesive may also have a storage modulus (G') higher than $0.3 \times 10^6$ at 70°C, as indicative of the temperature which the hot-melt has in the process (after initial cool-down due to heat exchange with the substrate onto which it has been applied) when it gets into contact with aperturing tooling.

A non-tacky behavior in the temperature range of about 40°C to about 70°C contributes to avoid contamination of the tooling, which can lead to frequent line stops.

EXAMPLES & DATA

The following examples, comparative examples, and data are provided to help illustrate the polymeric compositions. It will be appreciated that other modifications to the polymeric compositions described herein within the skill of those in the formulation art may be undertaken. All parts, percentages, and ratios herein are by weight unless otherwise specified.

Table 1

| Polymeric Composition | Tensile Strength at Yield (MPa) | Modulus of Elasticity (MPa) | Toughness [MJm$^{-3}$] | Yield Stress [MPa] |
|---|---|---|---|---|
| Licocene 2502[1] | 5.76 | 52.1 | 28.0 | 9.0 |
| Licocene 6502[2] | 4.94 | 235 | n.a. | n.a. |
| L-Modu S410[3] | 3.53 | 20.6 | 84.7 | 7.5 |
| REXtac RT-2830[4] | 2.06 | 19.4 | 22.9 | 2.9 |
| Bostik H2401[5] | 0.02 | 0.138 | n.a. | n.a. |
| Henkel DM3800[6] | 0.33 | 2.08 | n.a. | n.a. |
| 1: Available from Clariant 2: Available from Clariant 3: Available from Idemitsu 4: Available from REXtac LLC 5: Available from Bostik (comparative example) 6: Available from Henkel (comparative example) | | | | |

Table 1 provides measurements of the Tensile Strength at Yield, according to the Tensile Strength Test Method described herein, and the Modulus of Elasticity, according to the Modulus of Elasticity Test Method described herein for various bond areas comprising various polymeric compositions at various gsm amounts. Table 1 also provides measurements of the Tensile Strength at Yield, according to the Tensile Strength Test Method described herein, and the Modulus of Elasticity, according to the Modulus of Elasticity Test Method described herein. Bostik H2401 and Henkel DM3800 on the other hand are conventional formulated adhesives comprising significant amount of tackifiers and have been used to make absorbent hygiene products via conventional slot coating process.

ADDITIONAL DATA

A method and apparatus described above with reference to Figs. 1-10 was used to apply a polymeric composition consisting of Licocene 2502. The primary substrate on which the composition was applied was a 20 gsm Carded Nonwoven (ex. Dayuan, ref. number FJ206). The pattern on the roll consisted of rows of discrete square elements each having a 1 mm edge and each separated by a gap of 1 mm in cross direction, with each row separated by a gap of 7 mm in the machine-direction. A comb shim (1 mm ON 1 mm OFF in cross-direction) was also positioned in the die applicator with the ON areas corresponding to the discrete protruding square elements. A second substrate was used as a cover web (Fibertex 15 gsm Softblend IBNW) and was directly laminated on the substrate comprising the polymeric composition. The line speed as set at 150 m/min.
A high pattern quality (no smearing, very high resolution) was obtained (see UV picture shown in Fig. 14) as well as no blobbing recorded (over ~1 min run time).

TESTING METHODS

Tensile Strength at Yield Test Method

The Tensile Strength of a bonding material, a tackified adhesive composition, or a polymeric composition is determined using the Standard Test Method for Tensile Properties of Plastics, which consists of performing ASTM D638-14 with the following additional guidance. Ambient conditions are maintained at relative humidity of 50 $\pm$ 2 % and at 23 $\pm$ 1°C. Polymers and hot melt adhesive compositions are cast into a shape consistent with a Type IV "dogbone" as described in Fig 1 of ASTM D638-14 and allowed to cool to ambient conditions before conditioning the test specimens in accordance with Procedure A of ASTM Practice D618. The test proceeds with a crosshead speed of 50 mm/min.
Tensile Strength at Yield is calculated as described in section 11.2 of ASTM D638-14 and is reported as the "Tensile Strength at Yield" in units of megapascals (MPa) to the nearest 0.01 MPa.

Extensional Test Method

The Extensional Test Method is used to determine the Yield Stress and the Toughness for a specimen of a polymer composition. A thin film specimen formed of polymer composition is analyzed with a rotational rheometer fitted with a specialized fixture with counter rotating rollers, and the stress associated with extensional strain imparted is measured and recorded.

Instrumental setup

A rotational rheometer (ARES G2, TA Instruments, New Castle, DE, USA, or equivalent) is fitted with a fixture that has counter rotating cylindrical rollers specifically designed for the interrogation of extension deformation of films. An example of a suitable fixture is the Extensional Viscosity Fixture, or EVF (EVF, TA Instruments, or equivalent). The rheometer is further fitted with a forced-convection oven FCO (FCO, TA Instruments, or equivalent) and cooling system (ACS 2, TA Instruments, or equivalent) capable of controlling temperate from at least -50 to 250 °C to a within a tolerance of 0.5 °C.

Specimen preparation

Approximately 6 g $\pm$ 2 g of the polymer composition is placed in a circular polytetrafluoroethane (PTFE) bowl with a flat bottom (diameter of 60 mm $\pm$ 2 mm) and introduced into a vacuum oven held at 170°C. After 15 minutes at ambient pressure, the pressure is lowered to 10 mbar, and the polymer composition is subsequently held at 170°C and at 10 mbar for 45 minutes to remove air bubbles from the polymer composition. If 170°C is insufficient to melt the polymer compositions a temperature 30 $\pm$ 10 °C above the melting temperature of the polymer material composition is used. The polymer composition is removed from the vacuum oven and allowed to cool to ambient lab conditions (23 $\pm$ 2 °C) for 90 $\pm$ 30 minutes, at which point the polymer composition is removed from the PTFE bowl and placed between 2 sheets of siliconised paper (such as product number 114918, Mondi Group, Hilm, Austria, or equivalent). A metal shim 500 $\pm$ 30 $\mu$m in thickness is used in the heated press as a spacer to obtain a film thickness of 500 $\mu$m when pressed with a heated press at 90 °C for 60 seconds at a pressure sufficient to form a polymeric film. If 90 °C is insufficient to press a uniform flat film, a temperature approximately 10 $\pm$ 5 °C below the melting point of the sample material composition such that the sample material composition is in a semi-solid state is used. The film is stored at least 120 hours in the laboratory at 23 $\pm$ 2 °C prior to testing. From the film individual specimens for measurement are punched with a sample cutter to the final specimen dimensions of 20.0 mm by 10.0 mm by 500 $\mu$m.

Measurement

To secure the film to the cylinders of the EVF, the cylinders are heated to 50 °C for 90 $\pm$ 30 s in the forced-convection oven of the rheometer. After opening the oven, a specimen of polymer composition is briefly pressed onto the cylinders of the EVF to secure it to the cylinder surface. The specimen is placed perpendicular to the axis of rotation of the cylinders.

The specimen mounted on the EVF is then placed in the forced convection oven of the rheometer for thermal conditioning and is kept isothermal at 23 $\pm$ 1 °C for 300 $\pm$ 10 s. After this time has elapsed, the specimen is mechanically conditioned. To mechanically condition the specimen, the torque transducer is zeroed, and the sample is put under a pre-stretch rate of 0.001 s$^{-1}$ for 0.30 s and then allowed to relax for 60 s (in this method, all strain is expressed in terms of Hencky strain, also known as "true strain" or "logarithmic strain.").

The measurement is performed in the FCO oven at 23 °C $\pm$ 0.5 °C. The strain rate extension for the measurement is 1 s$^{-1}$, and the strain at maximum extension is 4.0. After measurement, the specimen is checked for rupturing. If it has ruptured, the location of the break is noted. If the rupture is approximately in the middle between the two cylinders of the EVF, the data collected are deemed acceptable. Otherwise, if the polymeric film break is at or close to the rotating cylinders, the results are discarded, and the measurement performed again on a replicate specimen.

Analysis

For the extensional stress calculation, a constant volume is assumed. From the raw torque versus angular displacement data recorded by the rheometer, extensional stress (in megapascals, or MPa) versus Hencky strain data are calculated. The data are plotted in semilogarithmic fashion with Hencky strain on the abscissa (linear scale) and extensional stress on the ordinate (logarithmic scale). A linear range is sought in this plot. If a linear range above a strain of 0.3 can be identified and this range can be fit with a positive slope with an $R^2$ value of 0.98 or greater, the value of the fitted line at a Hencky strain of zero (that is, the y-intercept), is defined as the Yield Stress, which is reported in MPa to the nearest kilopascal. Otherwise, the maximum value of extensional stress recorded during the measurement is reported as the Yield Stress, again reported in MPa to the nearest kilopascal.

The extensional stress (MPa) versus Hencky strain data calculated above are again plotted, but this time in linear fashion with Hencky strain on the abscissa (linear axis) and extensional stress on the ordinate (linear axis). The integral of extensional stress with strain (that is, the area under the extensional stress curve as a function of strain) is calculated from a strain of zero to the strain at which the sample ruptured (or, in the case it did not rupture during the measurement, to a strain of 4.0) and is reported as the Toughness, which is reported in units of megajoules per cubic meter, or MJ m$^{-3}$.

Oscillatory Rheometry Test Method

The Oscillatory Rheometry Test Method is used to measure the Storage Modulus G' and the Loss Factor of a polymer composition. A controlled-strain rotational rheometer (such as Discovery HR-3, TA Instruments, New Castle, DE, USA, or equivalent) capable of sample temperature control (using a Peltier cooler and resistance heater combination) with a precision equal to or exceeding 0.5°C over at least the range of -10 °C to 150 °C. The rheometer is operated in a parallel plate configuration with 20-mm stainless steel parallel-plate tooling.

A parallel plate gap of 1000 $\mu$m is initially used in the method. To compensate for thermal expansion of the tooling, the gap is set to 1000 $\mu$m, and a mapping of actual plate gap (as measured using a suitable standard test fluid) a function of temperature over the range -10 °C to 150 °C is performed. This mapping is then used throughout the determination of the Storage Modulus Parameter and the Loss Factor Parameter.

The rheometer is heated to 150 °C, the polymer composition is introduced in the rheometer, the gap is set to 1050 $\mu$m, excess protruding sample is trimmed, and the gap is then set to 1000 $\mu$m. (The axial force control of the rheometer is set to 0 N and be maintained within $\pm$ 0.1 N of force during the experiment, thereby thermal expansion/contraction of the sample itself is compensated by adjusting the gap in order to avoid overfilling or underfilling in addition to the abovementioned compensation of the tooling.) The rheometer is then allowed to cool to 130 °C, at which point the measurement commences with temperature ramped from 130 °C to -10 °C at a constant rate of cooling of 2 °C/min. The applied strain amplitude is 0.1%, and the frequency of oscillation is 1 Hz (that is, one cycle per second). The resulting oscillatory stress is recorded.

After this step, the sample temperature is set to 23 °C (temperature is ramped to this setpoint at a rate of 10 °C/min), and the sample is allowed to rest for 4.0 hours at 23 °C. At the end of this period, the temperature is set to -10 °C (temperature is ramped to this setpoint at a rate of 10 °C/min), the sample is equilibrated for 300 seconds at -10 °C, and a second oscillatory rheology measurement is conducted (0.1% strain, frequency of oscillation of 1 Hz) while temperature is ramped upward to 130 °C at a constant rate of increase of 2 °C/min.

From the first decreasing temperature sweep, the storage modulus G' is calculated and recorded at 37°C, and these values are reported in Pascals (Pa) to the nearest 1 Pa as the "Storage Modulus at 100 °C". From the first, decreasing temperature sweep, the loss factor (also known as tan delta) is calculated recorded at 100°C, and this dimensionless value is reported to the nearest hundredth as the "Loss Factor at 100°C". The storage modulus G' can also be calculated and recorded at different temperatures, for example 60°C.

Viscosity Test Method

The Viscosity of a viscous or semi-liquid composition is determined by performing ASTM D3236-15 with the following additional guidance. A Brookfield RVT viscometer with spindle SC 4-27 (Brookfield Engineering, Middleboro, MA, USA), or equivalent, is used. The sample temperature is maintained at $170.0 \pm 1.0$ °C, unless otherwise specified, throughout the measurement. The sample is preheated for 10 minutes and stirred with the measurement spindle for 30 min. The spindle is rotated at 20 rpm throughout the measurement. The resulting apparent viscosity, as described in section 10, is reported as the "viscosity" in units of millipascal-seconds to the nearest 100 mPa·s.

Modulus of Elasticity Test Method

The Modulus of Elasticity of a bonding material, a tackified adhesive composition, or a polymeric composition is determined using the Standard Test Method for Tensile Properties of Plastics, which consists of performing ASTM D638-14 with the following additional guidance. Ambient conditions are maintained at relative humidity of $50 \pm 2$ % and at $23 \pm 1$°C. Polymers and hot melt adhesive compositions are cast into a shape consistent with a Type IV "dogbone" as described in Fig 1 of ASTM D638-14 and allowed to cool to ambient conditions before conditioning the test specimens in accordance with Procedure A of ASTM Practice D618. The test proceeds with a crosshead speed of 50 mm/min.

The Modulus of Elasticity is also calculated as described in section 11.4 of ASTM D638-14 and is reported as the "Modulus of Elasticity" in units of MPa to the nearest 0.01 MPa.

**Claims**

1. A method for applying a polymeric composition (130), discharged in a fluid form from a slot die applicator (102) to a substrate (106), the slot die applicator (102) including a slot opening (114), a first lip (116) and a second lip (118), the slot opening (114) being located between the first lip (116) and the second lip (118); and the substrate (106) having a first surface (108) disposed opposite of a second surface (110) and an unconstrained caliper, Hs, the method comprising the steps of:

    - continuously advancing the substrate (106) in a machine direction;
    - engaging the substrate (106) with a substrate carrier (104) such that the first surface is in face-to-face contact with the substrate carrier and the second surface faces outward from the substrate carrier;
    - positioning the substrate carrier (104) adjacent the slot die applicator (102) to define a minimum distance, Hg, between the substrate carrier and the first lip (116) and the second lip (118) that is substantially the same or less than the unconstrained caliper, Hs, of the substrate (106);
    - advancing the second surface (110) of the substrate (106) past the first lip (116), the slot opening (114), and the second lip (118) of the slot die applicator (102) while the first surface (108) of the substrate (106) is disposed on the substrate carrier (104); and
    - discharging the polymeric composition (130) from the slot opening (114) of the slot die applicator (102) onto the second surface (110) of the substrate (106),

    wherein the polymeric composition comprises at least 50% of one or more polymers by weight of the polymeric composition and has a Tensile Strength at Yield of from 0.5 MPa to 10 MPa, as measured according to the Tensile Strength at Yield Test Method described herein.

2. A method according to claim 1, wherein the polymeric composition comprises less than 5 % of a tackifier, by weight of the polymeric composition.

3. A method according to claim 1 or 2, wherein the polymeric composition comprises at least one polymer selected from the group consisting of a propylene butene copolymer, a polypropylene homopolymer, a propylene ethylene copolymer, and mixtures thereof.

4. A method according to any of the preceding claims, wherein the polymeric composition has a Toughness of at least 25 MJ.m$^{-3}$, as measured according to the Extensional Test Method disclosed herein, and/or wherein the polymeric composition has a storage modulus (G') of higher than 300,000 Pa at 37°C, preferably of higher than 300,000 Pa at 60°C, wherein the G' is measured with the Oscillatory Rheometry Test Method described herein.

5. A method according to any of the preceding claims, wherein the substrate carrier is selected from a flat or curved plate (104'), a smooth roll, or a patterned roll (104), wherein the roll is optionally driven.

6. A method according to any of the preceding claims, wherein the polymeric composition is discharged from a slot die applicator (102) to a substrate (106) in a pattern, the method comprising the steps of:

   - continuously advancing the substrate (106) in a machine direction;
   - engaging the substrate (106) with a substrate carrier (104), such that the first surface (108) is in face-to-face contact with the substrate carrier (104) and the second surface (110) faces outward from the substrate carrier (104), wherein the substrate carrier (104) comprises a non-compliant support surface (162) and a pattern element (122), the pattern element including a pattern surface (126), wherein pattern element extends away from the non-compliant support surface to define a first minimum distance, R1, between the pattern surface (126) and the non-compliant support surface (162);
   - positioning the substrate carrier (104) adjacent the slot die applicator (102) to define a minimum distance, Hg, between the pattern surface (126) of the pattern element and the first lip (116) and the second lip (118) that is less than the unconstrained caliper, Hs, of the substrate (106);
   - advancing the second surface (110) of the substrate (106) past the slot die applicator while the first surface (108) of the substrate is disposed on the substrate carrier (104);
   - intermittently deflecting the pattern surface toward the non-compliant support surface (162) such to define a second minimum distance, R2, between the pattern surface (126) and the non-compliant surface (162), wherein R2 is less than R1, by advancing the substrate and the pattern element past the first lip (116), the slot opening (114), and the second lip (118) of the slot die applicator (102) while the first surface of the substrate is disposed on the substrate carrier (104); and
   - discharging the fluid from the slot opening (114) of the slot die applicator onto the second surface (110) of the substrate (106).

7. A method according to the preceding claim, wherein the substrate carrier (104) comprises a roller (120), and wherein the roller (120) comprises a base roll (160) having an outer circumferential surface that defines the non-compliant support surface (162), and wherein the base roll is adapted to rotate about an axis of rotation (105), wherein the pattern element protrudes radially outward from the axis of rotation (105).

8. A method according to claims 6 or 7, the substrate carrier further comprising a base layer of compliant material extending radially outward from the non-compliant support surface to define a base surface, and wherein the compliant pattern element includes a proximal end portion and a distal end portion, wherein the proximal end portion is connected with base surface, the pattern element extending radially outward from the base surface to the distal end portion, wherein the substrate carrier (104) further comprises a compliant base surface (124), wherein the pattern element protrudes from the compliant base surface to define a distance, Hp, between the pattern surface and the compliant base surface.

9. A method according to claim 8, wherein the substrate carrier is positioned adjacent the slot die applicator to define a minimum distance, Hg, between the pattern surface of the pattern element and the first lip and the second lip that is less than the unconstrained caliper, Hs, of the substrate; and wherein a sum of the distance, Hp, and distance, Hg, is greater than the unconstrained caliper, Hs, of the substrate.

10. A method according to claim 8, wherein the substrate carrier further comprises a compliant layer of material between the pattern element and the non-compliant support surface, and wherein the compliant layer of material defines the base surface.

11. A method according to any of the preceding claims, wherein the substrate is subject to a tension per unit of length at the point of application of less than 10 N/m.

12. A method according to any of the preceding claims, wherein a second substrate is applied onto the second surface of the substrate comprising the polymeric composition while the composition is still in fluid form so that the substrate and the second substrate are bonded to each other by the polymeric composition, preferably wherein the two substrates are nonwovens and are bonded by the polymeric composition according to a bonding pattern corresponding to an application pattern.

13. An absorbent article (250) comprising a substrate, in particular a nonwoven material, and a polymeric composition applied on the substrate according to a contact application, the polymeric composition having a Tensile Strength at Yield of from 0.5 MPa to 10 MPa according to the Tensile Strength Test Method described herein, wherein the application pattern does not consist solely of one or more stripes oriented in machine direction, in particular wherein the polymeric composition comprises less than 5 % of a tackifier, by weight of the polymeric composition and/or wherein the polymeric composition comprises at least one polymer selected from the group consisting of a propylene butene copolymer, a polypropylene homopolymer, a propylene ethylene copolymer, and mixtures thereof.

14. An absorbent article according to claim 13, wherein the application pattern comprises continuous and/or discrete pattern elements having a dimension in machine direction of less than 10 mm, in particular wherein the elements are discrete pattern elements.

15. An absorbent article, according to claim 13 or 14, wherein the application pattern comprises at least one cross-direction oriented stripe(s), in particular wherein the one or more cross-direction stripe(s) form one or more core end seal(s).

**Patentansprüche**

1. Verfahren zum Auftragen einer Polymerzusammensetzung (130), die in einer Fluidform von einem Schlitzdüsenapplikator (102) auf ein Substrat (106) ausgeschieden wird, wobei der Schlitzdüsenapplikator (102) eine Spaltöffnung (114), eine erste Lippe (116) und eine zweite Lippe (118) einschließt, wobei sich die Spaltöffnung (114) zwischen der ersten Lippe (116) und der zweiten Lippe (118) befindet; und das Substrat (106) eine erste Oberfläche (108), die gegenüber einer zweiten Oberfläche (110) und einer unbegrenzten Dicke, Hs, angeordnet ist, aufweist, das Verfahren umfassend die Schritte zum:

- ununterbrochenen Vorschieben des Substrats (106) in einer Maschinenrichtung;
- Ineingriffnehmen des Substrats (106) mit einem Substratträger (104), derart, dass die erste Oberfläche in direktem Kontakt mit dem Substratträger steht und die zweite Oberfläche nach außen von dem Substratträger zeigt;
- Positionieren des Substratträgers (104) benachbart des Schlitzdüsenapplikators (102), um einen Mindestabstand, Hg, zwischen dem Substratträger und der ersten Lippe (116) und der zweiten Lippe (118), der im Wesentlichen gleich oder kleiner als die unbegrenzte Dicke, Hs, des Substrats (106) ist, zu bestimmen;
- Vorschieben der zweiten Oberfläche (110) des Substrats (106) an der ersten Lippe (116), der Spaltöffnung (114), und der zweiten Lippe (118) des Schlitzdüsenapplikators (102) vorbei, während die erste Oberfläche (108) des Substrats (106) auf dem Substratträger (104) angeordnet ist; und
- Abscheiden der Polymerzusammensetzung (130) von der Spaltöffnung (114) des Schlitzdüsenapplikators (102) auf die zweite Oberfläche (110) des Substrats (106),

wobei die Polymerzusammensetzung wenigstens zu 50 Gew.-% ein oder mehrere Polymere der Polymerzusammensetzung umfasst und eine Zugfestigkeit bei einer Ausbeute von 0,5 MPa bis 10 MPa, gemessen gemäß der Zugfestigkeit bei dem hierin beschriebenen Ausbeuteprüfverfahren, aufweist.

2. Verfahren nach Anspruch 1, wobei die Polymerzusammensetzung weniger als zu 5 Gew.-% einen Klebrigmacher der Polymerzusammensetzung umfasst.

3. Verfahren nach Anspruch 1 oder 2, wobei die Polymerzusammensetzung wenigstens ein Polymer umfasst, das ausgewählt ist aus der Gruppe bestehend aus einem Propylen-Buten-Copolymer, einem Polypropylenhomopolymer, einem Propylen-Ethylen-Copolymer und Mischungen davon.

4. Verfahren nach einem der vorstehenden Ansprüche, wobei die Polymerzusammensetzung eine Zähigkeit von wenigstens 25 MJ.m$^{-3}$, gemessen gemäß dem hierin offenbarten Ausdehnungsprüfverfahren, aufweist und/oder wobei die Polymerzusammensetzung einen Speichermodul (G') von mehr als 300.000 Pa bei 37 °C, vorzugsweise von mehr als 300.000 Pa bei 60 °C aufweist, wobei der G' mit dem hierin beschriebenen oszillatorischen Rheometrieprüfverfahren gemessen wird.

5. Verfahren nach einem der vorstehenden Ansprüche, wobei der Substratträger aus einer flachen oder gekrümmten Platte (104'), einer glatten Walze oder einer gemusterten Walze (104) ausgewählt ist, wobei die Walze wahlweise angetrieben wird.

6. Verfahren nach einem der vorstehenden Ansprüche, wobei die Polymerzusammensetzung von einem Schlitzdüsenapplikator (102) auf ein Substrat (106) in einem Muster ausgeschieden wird, das Verfahren umfassend die Schritte zum:

- ununterbrochenen Vorschieben des Substrats (106) in einer Maschinenrichtung;
- Ineingriffnehmen des Substrats (106) mit einem Substratträger (104), derart, dass die erste Oberfläche (108) in direktem Kontakt mit dem Substratträger (104) steht und die zweite Oberfläche (110) von dem Substratträger (104) nach außen zeigt, wobei der Substratträger (104) eine nicht nachgiebige Stützoberfläche (162) und ein Musterelement (122) umfasst, wobei das Musterelement eine Musteroberfläche (126) einschließt, wobei sich das Musterelement von der nicht nachgiebigen Stützoberfläche weg erstreckt, um einen ersten Mindestabstand, R1, zwischen der Musteroberfläche (126) und der nicht nachgiebigen Stützoberfläche (162) zu bestimmen;
- Positionieren des Substratträgers (104) benachbart zu dem Schlitzdüsenapplikator (102), um einen Mindestabstand, Hg, zwischen der Musteroberfläche (126) des Musterelements und der ersten Lippe (116) und der zweiten Lippe (118), der kleiner als die nicht unbegrenzte Dicke, Hs, des Substrats (106) ist, zu bestimmen;
- Vorschieben der zweiten Oberfläche (110) des Substrats (106) an dem Schlitzdüsenapplikator vorbei, während die erste Oberfläche (108) des Substrats auf dem Substratträger (104) angeordnet ist;
- ununterbrochenes Ablenken der Musteroberfläche zu der nicht nachgiebigen Trägeroberfläche (162), derart, um einen zweiten Mindestabstand, R2, zwischen der Musteroberfläche (126) und der nicht nachgiebigen Oberfläche (162) zu bestimmen, wobei R2 kleiner als R1 ist, durch das Vorschieben des Substrats und des Musterelements an der ersten Lippe (116), der Spaltöffnung (114) und der zweiten Lippe (118) des Schlitzdüsenapplikators (102) vorbei, während die erste Oberfläche des Substrats auf dem Substratträger (104) angeordnet ist; und
- Ausscheiden des Fluids aus der Spaltöffnung (114) des Schlitzdüsenapplikators auf die zweite Oberfläche (110) des Substrats (106).

7. Verfahren nach dem vorstehenden Anspruch, wobei der Substratträger (104) eine Rolle (120) umfasst und wobei die Rolle (120) eine Basisrolle (160), die eine äußere Umfangsoberfläche aufweist, die die nicht nachgiebige Stützoberfläche (162) bestimmt, umfasst, und wobei die Basisrolle konzipiert ist, um sich um eine Drehachse (105) zu drehen, wobei das Musterelement von der Drehachse (105) radial nach außen vorsteht.

8. Verfahren nach den Ansprüchen 6 oder 7, der Substratträger ferner umfassend eine Basisschicht aus nachgiebigem Material, das sich von der nicht nachgiebigen Stützoberfläche radial nach außen erstreckt, um eine Basisoberfläche zu bestimmen, und wobei das nachgiebige Musterelement einen proximalen Endabschnitt und einen distalen Endabschnitt einschließt, wobei der proximale Endabschnitt mit der Basisoberfläche verbunden ist, wobei sich das Musterelement von der Basisoberfläche zu dem distalen Endabschnitt radial nach außen erstreckt, wobei der Substratträger (104) ferner eine nachgiebige Basisoberfläche (124) umfasst, wobei das Musterelement von der nachgiebigen Basisoberfläche vorsteht, um einen Abstand, Hp, zwischen der Musteroberfläche und der nachgiebigen Basisoberfläche zu bestimmen.

9. Verfahren nach Anspruch 8, wobei der Substratträger zu dem Schlitzdüsenapplikator benachbart angeordnet ist, um einen Mindestabstand, Hg, zwischen der Musteroberfläche des Musterelements und der ersten Lippe und der zweiten Lippe, der kleiner als die unbegrenzte Dicke, Hs, des Substrats ist, zu bestimmen; und wobei eine Summe des Abstands, Hp, und des Abstands, Hg, größer als die unbegrenzte Dicke, Hs, des Substrats ist.

10. Verfahren nach Anspruch 8, wobei der Substratträger ferner eine nachgiebige Materialschicht zwischen dem Musterelement und der nicht nachgiebigen Trägeroberfläche umfasst und wobei die nachgiebige Schicht des Materials die Basisoberfläche bestimmt.

11. Verfahren nach einem der vorstehenden Ansprüche, wobei das Substrat einer Zugspannung pro Längeneinheit an dem Auftragepunkt von weniger als 10 N/m ausgesetzt ist.

12. Verfahren nach einem der vorstehenden Ansprüche, wobei ein zweites Substrat auf die zweite Oberfläche des Substrats aufgetragen wird, umfassend die Polymerzusammensetzung, während die Zusammensetzung noch in Fluidform ist, sodass das Substrat und das zweite Substrat durch die Polymerzusammensetzung aneinander gebunden sind, vorzugsweise wobei die zwei Substrate Vliese sind und durch die Polymerzusammensetzung gemäß einem Klebemuster, das einem Auftragemuster entspricht, gebunden sind.

13. Absorptionsartikel (250), umfassend ein Substrat, insbesondere ein Vliesmaterial und eine Polymerzusammensetzung, die auf das Substrat gemäß einer Kontaktauftragung aufgetragen ist, wobei die Polymerzusammensetzung eine Zugfestigkeit bei der Ausbeute von 0,5 MPa bis 10 MPa gemäß dem hierin beschriebenen Zugfestigkeitsprüfverfahren aufweist, wobei das Auftragemuster nicht ausschließlich aus einem oder mehreren in der Maschinenrichtung ausgerichteten Streifen besteht, insbesondere wobei die Polymerzusammensetzung weniger als zu 5 Gew-% einen Klebrigmacher der Polymerzusammensetzung umfasst und/oder wobei die Polymerzusammensetzung wenigstens ein Polymer, das ausgewählt ist aus der Gruppe bestehend aus einem Propylen-Buten-Copolymer, einem Polypropylenhomopolymer, einem Propylen-Ethylen-Copolymer und Mischungen davon, umfasst.

14. Absorptionsartikel nach Anspruch 13, wobei das Auftragemuster ununterbrochene und/oder separate Musterelemente, die eine Abmessung in der Maschinenrichtung von weniger als 10 mm aufweisen, umfasst, insbesondere wobei die Elemente separate Musterelemente sind.

15. Absorptionsartikel nach Anspruch 13 oder 14, wobei das Auftragemuster wenigstens einen in einer Querrichtung ausgerichteten Streifen umfasst, insbesondere wobei der eine oder die mehreren in der Querrichtung ausgerichteten Streifen eine oder mehrere Kernenddichtung(en) ausbilden.

## Revendications

1. Procédé destiné à appliquer une composition polymère (130), émise sous une forme de fluide à partir d'un applicateur à filière plate (102) vers un substrat (106), l'applicateur à filière plate (102) comportant une ouverture plate (114), une première lèvre (116) et une seconde lèvre (118), l'ouverture plate (114) étant située entre la première lèvre (116) et la seconde lèvre (118) ; et le substrat (106) ayant une première surface (108) disposée à l'opposé d'une seconde surface (110) et une épaisseur non contrainte, Hs, le procédé comprenant les étapes consistant à :

   - faire avancer en continu le substrat (106) dans une direction de la machine ;
   - mettre en prise le substrat (106) avec un transporteur de substrat (104) de telle sorte que la première surface est en contact face à face avec le transporteur de substrat et la seconde surface est tournée vers l'extérieur à partir du transporteur de substrat ;
   - positionner le transporteur de substrat (104) adjacent à l'applicateur à filière plate (102) pour définir une distance minimale, Hg, entre le transporteur de substrat et la première lèvre (116) et la seconde lèvre (118) qui est sensiblement la même ou inférieure à l'épaisseur non contrainte, Hs, du substrat (106) ;
   - faire avancer la seconde surface (110) du substrat (106) au-delà de la première lèvre (116), de l'ouverture plate (114), et de la seconde lèvre (118) de l'applicateur à filière plate (102) alors que la première surface (108) du substrat (106) est disposée sur le transporteur de substrat (104) ; et
   - émettre la composition polymère (130) à partir de l'ouverture plate (114) de l'applicateur à filière plate (102) sur la seconde surface (110) du substrat (106),

   dans lequel la composition polymère comprend au moins 50 % d'un ou plusieurs polymères en poids de la composition polymère et a une résistance à la traction à la limite élastique allant de 0,5 MPa à 10 MPa, telle que mesurée selon le procédé de test de résistance à la traction à la limite élastique décrit ici.

2. Procédé selon la revendication 1, dans lequel la composition polymère comprend moins de 5 % d'un agent poisseux, en poids de la composition polymère.

**3.** Procédé selon la revendication 1 ou 2, dans lequel la composition polymère comprend au moins un polymère choisi dans le groupe constitué de copolymère de propylène-butène, homopolymère de polypropylène, copolymère de propylène-éthylène, et mélanges de ceux-ci.

**4.** Procédé selon l'une quelconque des revendications précédentes, dans lequel la composition polymère a une ténacité d'au moins 25 MJ.m$^{-3}$, telle que mesurée selon le procédé de test d'allongement décrit ici, et/ou dans lequel la composition polymère a un module de conservation (G') supérieur à 300 000 Pa à 37 °C, de préférence supérieur à 300 000 Pa à 60 °C, dans lequel le G' est mesuré avec le procédé de test de rhéométrie oscillatoire décrit ici.

**5.** Procédé selon l'une quelconque des revendications précédentes, dans lequel le transporteur de substrat est choisi parmi une plaque plate ou courbée (104'), un rouleau lisse, ou un rouleau à motif (104), dans lequel le rouleau est éventuellement entraîné.

**6.** Procédé selon l'une quelconque des revendications précédentes, dans lequel la composition polymère est émise à partir d'un applicateur à filière plate (102) vers un substrat (106) suivant un motif, le procédé comprenant les étapes consistant à :

- faire avancer en continu le substrat (106) dans une direction de la machine ;
- mettre en prise le substrat (106) avec un transporteur de substrat (104), de telle sorte que la première surface (108) est en contact face à face avec le transporteur de substrat (104) et la seconde surface (110) est tournée vers l'extérieur à partir du transporteur de substrat (104), dans lequel le transporteur de substrat (104) comprend une surface de support non souple (162) et un élément à motif (122), l'élément de motif comportant une surface de motif (126), dans lequel l'élément de motif s'étend loin de la surface de support non souple pour définir une première distance minimale, R1, entre la surface de motif (126) et la surface de support non souple (162) ;
- positionner le transporteur de substrat (104) adjacent à l'applicateur à filière plate (102) pour définir une distance minimale, Hg, entre la surface de motif (126) de l'élément de motif et la première lèvre (116) et la seconde lèvre (118) qui est inférieure à l'épaisseur non contrainte, Hs, du substrat (106) ;
- faire avancer la seconde surface (110) du substrat (106) au-delà de l'applicateur à filière plate alors que la première surface (108) du substrat est disposée sur le transporteur de substrat (104) ;
- dévier de façon intermittente la surface de motif en direction de la surface de support non souple (162) de façon à définir une seconde distance minimale, R2, entre la surface de motif (126) et la surface non souple (162), dans lequel R2 est inférieure à R1, en faisant avancer le substrat et l'élément de motif au-delà de la première lèvre (116), de l'ouverture plate (114), et de la seconde lèvre (118) de l'applicateur à filière plate (102) alors que la première surface du substrat est disposée sur le transporteur de substrat (104) ; et
- émettre le fluide à partir de l'ouverture plate (114) de l'applicateur à filière plate sur la seconde surface (110) du substrat (106).

**7.** Procédé selon la revendication précédente, dans lequel le transporteur de substrat (104) comprend un rouleau (120), et dans lequel le rouleau (120) comprend un rouleau de base (160) ayant une surface circonférentielle externe qui définit la surface de support non souple (162), et dans lequel le rouleau de base est adapté pour être en rotation autour d'un axe de rotation (105), dans lequel l'élément de motif fait saillie de façon radiale vers l'extérieur à partir de l'axe de rotation (105).

**8.** Procédé selon les revendications 6 ou 7, le transporteur de substrat comprenant en outre une couche de base de matériau souple s'étendant de façon radiale vers l'extérieur à partir de la surface de support non souple pour définir une surface de base, et dans lequel l'élément de motif souple comporte une partie d'extrémité proximale et une partie d'extrémité distale, dans lequel la partie d'extrémité proximale est reliée à la surface de base, l'élément de motif s'étendant radialement vers l'extérieur à partir de la surface de base jusqu'à la partie d'extrémité distale, dans lequel le transporteur de substrat (104) comprend en outre une surface de base souple (124), dans lequel l'élément de motif fait saillie à partir de la surface de base souple pour définir une distance, Hp, entre la surface de motif et la surface de base souple.

**9.** Procédé selon la revendication 8, dans lequel le transporteur de substrat est positionné adjacent à l'applicateur à filière plate pour définir une distance minimale, Hg, entre la surface de motif de l'élément de motif et la première lèvre et la seconde lèvre qui est inférieure à l'épaisseur non contrainte, Hs, du substrat ; et dans lequel une somme de la distance, Hp, et de la distance, Hg, est supérieure à l'épaisseur non contrainte, Hs, du substrat.

**10.** Procédé selon la revendication 8, dans lequel le transporteur de substrat comprend en outre une couche souple

EP 3 892 246 B1

de matériau entre l'élément de motif et la surface de support non souple, et dans lequel la couche souple de matériau définit la surface de base.

11. Procédé selon l'une quelconque des revendications précédentes, dans lequel le substrat est soumis à une tension par unité de longueur au niveau du point d'application inférieure à 10 N/m.

12. Procédé selon l'une quelconque des revendications précédentes, dans lequel un second substrat est appliqué sur la seconde surface du substrat comprenant la composition polymère alors que la composition est toujours sous forme de fluide de sorte que le substrat et le second substrat sont liés l'un à l'autre par la composition polymère, de préférence dans lequel les deux substrats sont des nontissés et sont liés par la composition polymère selon un motif de liaison correspondant à un motif d'application.

13. Article absorbant (250) comprenant un substrat, en particulier un matériau nontissé, et une composition polymère appliquée sur le substrat selon une application de contact, la composition polymère ayant une résistance à la traction à la limite élastique allant de 0,5 MPa à 10 MPa selon le procédé de test de résistance à la traction décrit ici, dans lequel le motif d'application ne consiste pas uniquement en une ou plusieurs bandes orientées dans la direction de la machine, en particulier dans lequel la composition polymère comprend moins de 5 % d'un agent poisseux, en poids de la composition polymère et/ou dans lequel la composition polymère comprend au moins un polymère choisi dans le groupe constitué de copolymère de propylène-butène, homopolymère de polypropylène, copolymère de propylène-éthylène, et mélanges de ceux-ci.

14. Article absorbant selon la revendication 13, dans lequel le motif d'application comprend des éléments de motif continus et/ou discrets ayant une dimension dans la direction de la machine inférieure à 10 mm, en particulier dans lequel les éléments sont des éléments de motif discrets.

15. Article absorbant, selon la revendication 13 ou 14, dans lequel le motif d'application comprend au moins une bande(s) orientée(s) dans la direction croisée, en particulier dans lequel l'une ou plusieurs bande(s) dans la direction croisée forment un ou plusieurs joint(s) d'extrémité d'âme.

28

**Fig. 1**

Fig. 1A

Fig. 1B

**Fig. 1C**

**Fig. 1D**

**Fig. 2A**

EP 3 892 246 B1

Fig. 2B

Fig. 3A

**Fig. 3B**

EP 3 892 246 B1

**Fig. 2C**

**Fig. 3C**

Fig. 4

Fig. 4A2

Fig. 4A1

Fig. 4B2

Fig. 4B1

**Fig. 4C1**

**Fig. 4C2**

EP 3 892 246 B1

**Fig. 5**

**Fig. 6A**

Fig. 6B

**Fig. 6C**

Fig. 6D

**Fig. 6E**

Fig. 7

Fig. 8

**Fig. 9**

Fig. 10A

Fig. 10B

EP 3 892 246 B1

Fig. 11

Fig. 12

Fig. 13

Fig. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2140296811 A1, Bunnelle
- US 20160053149 A1, Herrlich
- US 20200108167 A1
- WO 2014085063 A, Brown
- US 7056386 B
- US 20080132865 A1
- US 7432413 B
- US 20070142798 A
- US 20070287983 A
- US 20070219521 A1, Hird
- US 20110139658 A1, Hird
- US 20110139657 A1, Hird
- US 20110152812 A1, Hird
- US 20110139662 A1, Hird
- US 20110139659 A1, Hird
- US 20060048880 A1
- US 20080215166 A1
- US 20100051166 A1
- US 20110137274 A1
- EP 0745368 A1
- US 5302675 A
- US 5723546 A